# EUROPEAN PATENT APPLICATION

(11) **EP 1 614 394 A2**
(43) Date of publication of application: **11.01.2006**
(21) Application number: 05254131.5
(22) Date of filing: 30.06.2005
(51) Int. Cl.: A61B 19/00, F21V 33/00

(54) **Support system for dentistry**

(30) Priority: 02.07.2004 US 585224 P; 25.08.2004 US 604577 P; 26.11.2004 US 631267 P; 04.01.2005 US 641462 P; 04.01.2005 US 641469 P; 04.01.2005 US 641461 P; 04.01.2005 US 641468 P; 26.01.2005 US 647725 P; 26.01.2005 US 647723 P; 26.01.2005 US 647580 P; 26.01.2005 US 647612 P; 26.01.2005 US 647593 P; 03.03.2005 US 658517 P; 22.03.2005 US 664696 P; 25.03.2005 US 594297 P; 30.03.2005 US 594327 P
(71) Applicant: Discus Dental Impressions Inc., Culver City, California 90232 (US)
(72) Inventor: Rose, Eric, Paul, Culver City, California 90232 (US); Grambush, Douglas H., Corona Del Mar, California 92625 (US); Hayman, Robert, Culver City, California 90232 (US); Kennedy, Brian, Claremont, CA 91711 (US); Orloff, Marc, Altadena, CA 91001 (US); Quan, Nancy N., Culver City, California 90232 (US); Rosenblood, Ken, Culver City, California 90232 (US); Vu, Dac, Irvine, California 92606 (US); Danta, Randall, Tustin, California 92780 (US); Quan, Christopher, Ng, Quincy, Massachussets 02140 (US)
(74) Representative: Lucas, Phillip Brian

(57) **Abstract**

A dental lamp boom hinge includes a pivot supporting a dental lamp head and a base supporting the pivot where the pivot may be rotated about a substantially vertical axis with respect to the base. The rotational movement between the pivot and the base is damped with a damping device. The damping device in one embodiment is adjustable to provide a variable level of damping.

## Description

### Cross-Reference to Related Applications

This application claims the benefit of U.S. provisional patent applications Serial No. 60/585,224, filed July 2, 2004, entitled "Dental Light Devices With Phase Change Heat Sink"; 60/641,462, filed January 4, 2005, entitled "Boom Hinge For A Dental Lamp"; 60/647,725, filed January 26, 2005, entitled "Automatic Control for a Dental Whitening Lamp"; 60/647,723, filed January 26, 2005, entitled "Boom Hinge For A Dental Lamp"; 60/658,517, filed March 3, 2005, entitled "Apparatus and Method For Radiation Spectrum Shifting in Dentistry Application"; 60/641,469, filed January 4, 2005, entitled "Lamp For Dentistry Applications"; 60/647,580, filed January 26, 2005, entitled "Light Guide For Dental Whitening Lamp"; 60/641,461, filed January 4, 2005, entitled "Support Structure For A Dental Lamp"; 60/641,468, filed January 4, 2005, entitled "Light Guide For A Dental Whitening Lamp"; 60/647,612, filed January 26, 2005, entitled "Light Path Apparatus For A Dental Lamp"; 60/647,593, filed January 26, 2005, entitled "Support Structure For A Dental Lamp"; U.S. design patent applications serial no. 29/220,642, filed January 4, 2005, entitled "Lamp For Dentistry Applications"; 29/220,680, filed January 4, 2005, entitled "Light Guide For Dentistry Applications"; 29/220,679, filed January 4, 2005, entitled "Power Pack For Dentistry Applications"; 29/220,712, filed January 4, 2005, entitled "Support Structure For A Lamp For Dentistry"; 29/XXX,XXX filed on June 22, 2005 entitled, "Support Structure For Dental Applications; 29/XXX,XXX filed on June 22, 2005 entitled, "Support Structure for Dental Applications"; U.S. provisional applications Serial No. 60/604,577, filed August 25, 2004, entitled "Lip Retractors"; 60/594,297, filed March 25, 2005, entitled "Curing Light Having A Detachable Tip"; 60/631,267, filed November 26, 2004, entitled "Curing Light Having A Reflector"; 60/594,327, filed on March 30, 2005, entitled, "Curing Light"; and 60/664,696, filed March 22, 2005, entitled "Curing Light Having A Detachable Tip"; the contents of all of which are hereby incorporated by reference.

The present application is a continuation-in-part of the following U.S. design applications No.: 29/220,642, filed January 4, 2005, entitled "Lamp For Dentistry Applications"; 29/220,680, filed January 4, 2005, entitled "Light Guide For Dentistry Applications"; 29/220,679, filed January 4, 2005, entitled "Power Pack For Dentistry Applications"; 29/220,712, filed January 4, 2005, entitled "Support Structure For A Lamp For Dentistry"; 29/XXX,XXX filed on June 22, 2005 entitled, "Support Structure For Dental Applications; 29/XXX,XXX filed on June 22, 2005 entitled, "Support Structure for Dental Applications"; all of which are incorporated herein by reference.

The present application includes claims that may be related to the claims of co-pending United States patent applications, No. 10/XXX,XXX, to be concurrently filed, entitled "Dental Light Devices Having an Improved Heat Sink"; 10/XXX,XXX, to be concurrently filed, entitled "Voice Alert System for Dentistry Applications"; 10/XXX, XXX, to be concurrently filed, entitled "Retracting Devices"; 10/XXX,XXX, to be concurrently filed, entitled "Curing Light Capable of Multiple Wavelengths"; 10/XXX,XXX, to be concurrently filed, entitled "Curing Light"; 10/XXX, XXX, to be concurrently filed, entitled "Illumination System for Dentistry Applications"; and 10/XXX,XXX, to be concurrently filed, entitled "Light Guide for Dentistry Applications"; 10/XXX,XXX, to be concurrently filed, entitled "Automatic Control For Dental Applications"; the contents of all of which are hereby incorporated by reference.

### Field of the Invention

The present invention relates to dental equipment and more particularly to positioning apparatus for dental equipment.

### Background

The practice of dentistry remains highly labor-intensive. Moreover, the labor involved is highly skilled. Although some activities can be delegated and others automated, many of the important activities in dentistry must still be performed by a dentist. Whether a dental procedure is performed by a dentist or by other dental professional, the dentist or other dental professional must operate in a time-efficient manner in order to provide desired dental services at a sustainable cost.

This need for efficient operation is reflected in the design of the dental office. An adjustable dental chair allows for positioning of a patient in an orientation that permits easy access to the patients mouth. Dental instruments are provided in pre-packaged kits adapted for use in particular dental procedures. Storage cabinetry for materials and dental tools are placed in proximity to the patient for ready access by the dentist and/or his or her assistant.

In addition, work trays and shelves for supporting dental instruments and work lights for illuminating the patients' mouth are provided with adjustable support structures that are adapted to keep the instruments within easy reach and the work area well illuminated.

It is known to use a variety of articulated support structures in the context of a dental office. Examples of these structures are shown in United States Patent Numbers 4,013,328 to Wolf, 4,097,919 to Bobrick et al., 4,260,376 to Litel et al., 4,332,557 (reissued as 31548 to Watanabe, 4,437,144 to Guenther, 4,494,177 to Matthews, 4,581,698 to Oram, 4,934,933 to Fuchs, 5,497,295 to Gehly, 5,803,905 to Allred et al., 6,213,671 to Chang et al., 6,361,320 to Yarborough, 6,543,914 to Sander and 6,568,836 to Wahl. The disclosures of the foregoing patents are herewith incorporated by reference in their entirety.

The support structures described in these references include a variety of articulating joints adapted to provide adjustable spatial positioning of a load, such as a lamp or instrument tray. For example, US Patent Number 4,907,919 shows a track-mounted illumination system with a telescoping boom. U.S. Patent Number 6,543,914 shows a boom and strut arrangement mutually coupled to a gas pressure spring for supporting a surgical microscope and lamp. U.S. reissue patent number 31548 shows a dental operatory system in which a dental operatory lamp is suspended from a pivotable bracket, and U.S. Patent Number 4,437,144 shows a height adjustable support arm with a parallelogram linkage.

These afore-mentioned load-supporting systems offers certain characteristics that may be beneficial in the context of a particular use. They show by their variety, the importance of the problem of effective load positioning in allowing economically efficient dental activities.

### Summary of the Invention

In light of the background discussed above, the present invention is related to improving the efficiency of operations in a dental office.

The present invention includes a support system for dentistry applications adapted to support a variety of dental process equipment and ancillary dental equipment including, for example, a dental whitening system, a dental curing system, a dental examination system, a dental viewing and cleaning instrument; an imaging equipment; an X-ray equipment, a root canal apex locator, or similar, or combinations thereof.

The support system includes a mast and boom system, with the boom being pivotally mounted on a mast about a location spaced away from its ends. The boom includes at least one formation adapted for engaging with at least one corresponding inter-engaging formation towards one end of the mast to form the pivot mount.

In one aspect, the boom includes at least one formation towards one end, adapted for inter-engaging at least one formation of a dental instrument or equipment; and at least one formation towards a second end adapted for engaging a counterbalancing object or weight including at least one correspondingly inter-engaging formation, for counterbalancing the dental instrument or equipment and permitting the dental instrument or equipment to be balanced in a series of varying positions.

In one embodiment, the invention includes a support system adapted to support a plurality of dental instrument or equipment adapted for performing a varying series of dental processes including at least one formation for inter-engaging the corresponding formation on the boom, where the plurality of instrument or equipment may be adapted for interchangeable connection with the support system by varying the counter-balancing object or weight.

In one aspect, the support system may be adapted to cooperate in a novel fashion with novel and/or conventional equipment to establish and maintain respective position of processing equipment and a patient's mouth, through the use of, for example, a reference device and/or a spacer.

In another aspect, the support system provides an integrated system for the support, presentation and operation of various dental processing equipment, individually or concurrently.

In another embodiment, a system according to the invention includes an organized storage system for storing and retaining interchangeable process equipment when not in use.

In a further embodiment, the support system includes an integrated power and control module referred to, for example, as a power pack. The power pack may be adapted to provide power such as electrical power to the one or more dental instrument or equipment adapted to be supported by the support system of the present invention. The power pack includes at least one formation for inter-engaging at least one corresponding formation of the mast spaced away from the ends.

In one aspect, the power pack may be adapted to provide control functionality including, for example, operative control communications between the power pack and one or more dental processing apparatus.

In another aspect, the power pack may be adapted to provide intelligent communications with one or more dental processing apparatus such that an operative control communications between the power pack and the dental processing apparatus may be adapted to include communication features appropriate to a particular processing apparatus.

In a further aspect, the power pack may include user interface components adapted to receive control inputs from an operator and provide status and control feedback to the operator.

In other aspects, the features of the support system of the present invention may include desirable ergonomics, transportability, strength, optimal weight, ease of assembly, storability, maintainability, adjustability or positionability, one or more of which may contribute in varying degrees to the efficacy of resulting dental processing.

The present invention also provides a support system that is unobtrusive, enabling an office to be equipped with a number of such support systems, thus improving the efficiency of operations in a dental office.

The present invention further includes a boom hinge for allowing easy adjustment of any dental equipment adapted to be mounted on the support system, for example, a dental whitening illumination source. In various aspects, the boom hinge may be adapted to provide equipment positioning suitable for a wide variety of dental patients and dental professionals. In other aspects, a boom hinge according to the invention may be readily adjusted to allow a particular spatial positioning of any dental equipment, and once positioned, to hold the equipment substantially fixedly in space until a further adjustment of equipment position is desired.

In one embodiment, the invention includes a boom hinge for a dental whitening lamp having a pivot, a yoke with two sides, a shaft, and at least one internally threaded knob adapted to compress the two sides of the yoke onto the pivot.

In another embodiment, the invention further includes a plug to receive the shaft, for the pivot to rotate with respect to the plug.

In another embodiment, the invention includes a device for damping the rotational motion of the pivot. In one aspect, the device for damping may be a friction washer disposed between the pivot and the plug. In another aspect, the device for damping includes a friction pad disposed between the pivot shaft and the plug.

In a further embodiment, a spanner plug may be adapted to push the pivot shaft against the friction pad. In one aspect of this embodiment, the spanner plug may be adjustable thereby providing adjustable damping of the rotational movement of the pivot with respect to the plug. In another aspect of this embodiment, a spring may be adapted to push the friction pad against the shaft.

In yet another embodiment of the invention, the shaft includes a damping device. In one aspect of this embodiment, friction rings may be mounted to the shaft.

These and other advantages and features of the invention will be more readily understood in relation to the following detailed description of the invention, which is provided in conjunction with the accompanying drawings.

### Brief Description of the Drawings

FIG. 1 shows, in perspective view, a support structure for dentistry equipment according to one embodiment of the invention;

FIG. 2 shows, in sectional elevation, various aspects of a dentistry support structure;

FIG. 3a-3d show various aspects of a dentistry support structure;

FIG. 4 shows a support structure for dentistry applications according to another embodiment of the invention;

FIG. 5 shows, in perspective view, a base adapted for inclusion in a dentistry support structure;

FIG. 6 shows, in perspective view, a counterweight mounting shaft for a support structure for dentistry equipment;

FIG. 7 shows, in perspective view, a boom joint for a dentistry support structure;

FIG. 8 shows, a boom joint in side elevation;

FIG. 9 shows a cross-sectional view of a boom joint according to one embodiment of the invention;

FIG. 10 shows a perspective view of a boom joint pivot according to one embodiment of the invention;

FIG. 11 shows a perspective view of a boom hinge yoke according to one embodiment of the invention;

FIG. 12 shows a boom joint horizontal shaft according to one embodiment of the invention;

Figs. 13a and 13b show boom joint washers according to respective embodiments of the invention;

FIG. 14 shows, in perspective view, a boom joint knob according to one embodiment of the invention;

FIG. 15 shows, in perspective view, a boom joint according to one embodiment of the invention;

FIG. 16 shows, in ventral perspective view, a mast top plug according to one embodiment of the invention;

FIG. 17 shows a top view of a mast top plug according to one embodiment of the invention;

FIG. 18 shows, in cross-section, a mast top plug according to one embodiment of the invention;

FIG. 19 shows, in assembly view, a mast, boom and boom joint according to one embodiment of the invention;

FIG. 20 shows, in cross-section, a boom joint according to a further embodiment of the invention;

FIG. 21 shows, in cross-section, a boom joint according to a further embodiment of the invention;

FIG. 22 shows, in cross-section, a boom joint according to a further embodiment of the invention;

FIG. 23 shows a boom joint shaft according to one embodiment of the invention;

FIG. 24 shows a table of empirical values illustrating a functional relationship between patient push out force, joint torque, spring force and spring deflection according to one embodiment of the invention;

FIG. 25 shows, in graphical form, a functional relationship between spring deflection and patient push out force according to one embodiment of the invention;

FIG. 26 shows, in graphical form, a functional relationship between spring force and spring displacement according to one embodiment of the invention;

FIG. 27 shows, in elevation, a pivot and mast top plug assembly according to one embodiment of the invention;

FIG. 28 shows, in cross-section, a pivot and mast top plug assembly according to one embodiment of the invention;

FIG. 29 shows, in ventral perspective view, a pivot according to one embodiment of the invention;

FIG. 30 shows, in ventral perspective view, a vertical pivot shaft according to one embodiment of the invention;

FIG. 31 shows, in cross-section, a vertical pivot shaft according to one embodiment of the invention;

FIG. 32 shows, in ventral perspective view, a vertical pivot shaft according to another embodiment of the invention;

FIG. 33 shows, in cross-section, a vertical pivot shaft according to another embodiment of the invention;

FIG. 34 shows, in top view, a spanner nut according to one embodiment of the invention;

FIG. 35 shows, in side view, a spanner nut according to one embodiment of the invention;

FIG. 36 shows, in ventral perspective view, a bushing according to one embodiment of the invention;

FIG. 37 shows, in top view, a spanner nut according to another embodiment of the invention;

FIG. 38 shows, in ventral perspective view, a mast-top plug according to one embodiment of the invention;

FIG. 39 shows, in cross-section, a mast-top plug according to one embodiment of the invention;

FIG. 40 shows, in top view, a mast-top plug according to another embodiment of the invention;

FIG. 41 shows, in assembly view, a mast-top plug and pivot assembly according to another embodiment of the invention;

FIG. 42 shows, in perspective view, various components of a ball joint according to one embodiment of the invention;

FIG. 43 shows, in exploded perspective view, a forward assembly for a dentistry equipment support structure boom;

FIG. 44 shows, in sectional perspective view, components of a ball joint according to one embodiment of the invention;

FIG. 45 shows, in perspective view, a nut plate and spring assembly according to one embodiment of the invention;

FIG. 46 shows, in ventral perspective view, a ball cup according to one embodiment of the invention;

FIG. 47 shows, in perspective view, a ball cup according to another embodiment of the invention;

FIG. 48 shows, in perspective view, a ball joint assembly according to another embodiment of the invention;

FIG. 49 shows, in perspective view, a ball joint knob according to one embodiment of the invention;

FIG. 50 shows a support structure for dentistry equipment including a dental whitening lamp according to one embodiment of the invention;

FIG. 51 shows a support structure for dentistry equipment including a dental whitening lamp according to one embodiment of the invention;

FIG. 52 shows a portion of a support structure for dentistry equipment including a dental whitening lamp according to one embodiment of the invention;

FIG. 53 shows a device for coupling a patient's lips to a dental whitening lamp, and hence to a support structure for dentistry equipment according to one embodiment of the invention;

FIG. 54 shows a support structure for dentistry equipment including an endoscopic device such as an endoscopic tooth planer according to one embodiment of the invention;

FIG. 55 shows a support structure for dentistry equipment including an endodontic apex locator device according to one embodiment of the invention;

FIG. 56 shows a portion of a support structure for dentistry equipment including an x-ray film support structure according to one embodiment of the invention;

FIG. 57 shows a portion of a support structure for dentistry equipment including an electronic x-ray imaging sensor support structure according to one embodiment of the invention;

FIG. 58 shows a support structure for dentistry equipment including an ultrasonic imaging device according to one embodiment of the invention;

FIG. 59 shows a dental composition tray for use with a support structure according to principles of the invention.

### Detailed Description of the Invention

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

All publications mentioned herein are incorporated herein by reference for the purpose of describing and disclosing, for example, the designs and methodologies that are described in the publications which might be used in connection with the presently described invention. The publications listed or discussed above, below and throughout the text are provided solely for their disclosure prior to the filing date of the present application. Nothing herein is to be construed as an admission that the inventors are not entitled to antedate such disclosure by virtue of prior invention.

The detailed description set forth below is intended as a description of the presently preferred device provided in accordance with aspects of the present invention and is not intended to represent the only forms in which the present invention may be practiced or utilized. Rather, it is to be understood, that the same or equivalent functions and components may be accomplished by different embodiments that are also intended to be encompassed within the spirit and scope of the invention.

The support system of the present invention may be adapted to support a wide variety of equipment for dentistry. For example, the support structure of the present invention is adapted to support Chemical activation equipment such as dental whitening and dental curing radiation sources; dental imaging equipment such as x-ray positioning equipment, x-ray sensing equipment including film and electronic sensors, endoscopic imaging equipment, ultrasonic imaging equipment, and various other imaging equipment such as now exists or may come to exist in the field of dentistry.

The support system includes a boom, a mast, and a base in, for example, a modular form.

The boom includes at least one formation spaced away from its ends, adapted for inter-engaging a corresponding formation towards one end of the mast for pivotally mounting the boom to the mast, when the mast and the boom become apposed.

The boom includes at least one formation towards one end, adapted for inter-engaging a corresponding formation on a dental instrument or equipment, for mounting the dental instrument to the boom, when the boom and the instrument become apposed.

The boom further includes at least one formation towards a second end, adapted for inter-engaging a corresponding formation of a counter-balancing object or weight, when the boom and object become apposed.

The mast includes at least one formation spaced away from its ends, adapted for inter-engaging a corresponding formation on a power pack when the mast and the power become apposed.

The mast includes at least one formation towards a second end, adapted for inter-engaging a corresponding formation of a support base when the mast and the support base become apposed.

The word formation as used herein in relation to the reference device, spacer, the lamp system, the mast, the boom, the power pack, the base and the counter-balance object or weight or any other components of the support system refers to the portion of one component which is shaped to inter-fit with a corresponding part of an adjoining component. It includes portions of the above listed components which may be shaped by molding, casting, machining, or any other appropriate method, or portions which are formed separately and then subsequently assembled.

Suitable inter-engaging formations include, but is not limited to, tongues and grooves, posts and sockets, swingable hooks and sockets, resilient clips and sockets, tongue or wing-like members and slots, ball and cavity, ball and socket, screw and washer, depressions and protrusions, channels and rods or cables, and so on, some of which are more specifically exemplified in detail below.

The light system of the present invention may be easily aligned to a subject and is ergonomically compatible for both right-handed and left-handed users. Further, the pieces of the light system are separable and modular, as mentioned above, so that the light system is easy to assemble, disassemble, pack, ship or transport. In addition, individual pieces or modules may be sent in for repair or for updating.

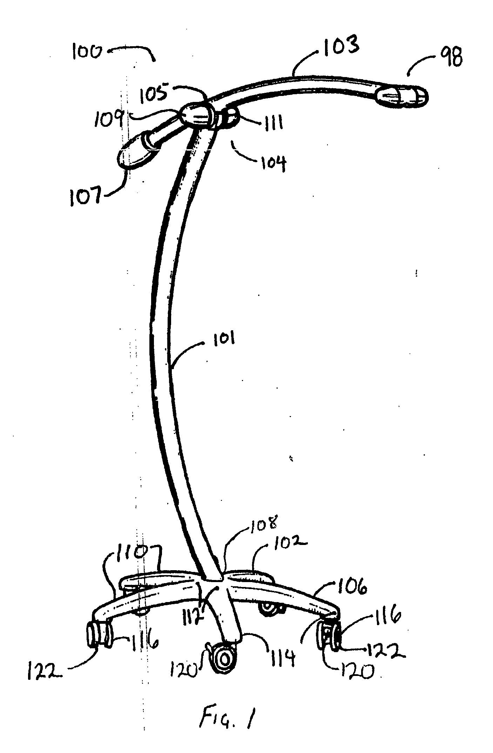

FIG. 1 shows a support structure 100 for dentistry equipment according to one embodiment of the invention. As illustrated, the support structure 100 includes a base 102 and an articulated support member 104. According to one embodiment of the invention, the base includes a body portion 106. The base 102 is adapted to be coupled to the articulated support member 104 by receiving a coupling feature 108 (see FIG. 3a below) of the articulated support member 104. The coupling feature 108 is adapted to substantially fixedly couple the articulated support member 104 to the body portion 106.

In one embodiment, the articulated support member includes a mast 101 and a boom 103. The mast 101 and boom 103 are coupled to one another by a boom joint 105 that includes boom joint knobs 109, 111.

In the embodiment of FIG. 1, an anterior end of the boom 103 includes a formation such as an instrument or apparatus-coupling feature 98. Although a wide variety of apparatus-coupling features are within the scope of the invention, the illustrated apparatus-coupling feature is a ball joint.

In various embodiments, the boom 103 of the support structure 100 includes a second formation, such as a boom-control feature adapted to offset a gravitational torque produced by a load mass being coupled to the apparatus-coupling feature. For example, in the illustrated embodiment, a posterior end of the boom 103 includes a boom counterweight 107. One of skill in the art will appreciate, however, that a wide variety of boom-control features, such as for example torsion springs and tension springs, are applicable to control of the boom 103, and are within the scope of the invention.

In one embodiment, as illustrated, the body portion 106 includes a plurality of beams 110 disposed in a radial orientation with respect to one another, such that a respective plurality of proximal ends 112 of the beams 110 are disposed proximate to the coupling feature 108, and a respective plurality of distal ends 114 of the beams 110 are disposed distal to the coupling feature 108. In various embodiments, the beams 110 and/or the body portion 106 may consist of a single integral member, or may be an assembly of discrete components.

According to one embodiment of the invention, as illustrated, the base 102 includes five beams 110 arranged radially in a substantially symmetrical, substantially coplanar, substantially horizontal orientation. A different number of beams is also envisioned.

In another embodiment of the invention, the body portion 106 may include a component having a disk shape, a hemispherical shape, a truncated hemispherical shape, a hemi-ellipsoid shape, a truncated hemi-ellipsoid shape, a conical shape, a truncated conical shape, and a wide variety of other shapes according to the various requirements, including functional and aesthetic requirements, of a particular embodiment.

In a further aspect, according to one embodiment of the invention, the base 102 includes a bearing device adapted to facilitate motion of the dental equipment support structure 100 with respect to a supporting surface, such as a floor. According to one embodiment, as illustrated, the bearing device includes a plurality of caster wheels 116. In one embodiment, the plurality of caster wheels is disposed at respective ends 114 of the plurality of beams 110. In another embodiment, the caster wheels are disposed adjacent a periphery of a disk shaped base.

In still another embodiment of the invention, the plurality of caster wheels 116 includes a respective plurality of braking devices. In a further aspect, each of the plurality of braking devices includes a discrete activation lever 120. In another embodiment of the invention, a single, common activation device is adapted to engage each of the plurality of braking devices.

In still another embodiment of the invention, a single common braking device is used. For example, according to one embodiment of the invention, base 102 includes a brake shoe mechanism adapted to urge a break shoe downwardly from adjacent a lower surface of the body 106 towards a floor, such that when the break shoe engages the floor, a frictional force between a lower surface of the break shoe and the floor inhibits lateral motion, with respect to the floor, of the support structure 100.

In a further aspect, according to one embodiment of the invention, the plurality of caster wheels 116 includes a respective plurality of tires 122. In various embodiments, these tires may be formed of various materials used alone or in combination. Such materials include, for example, elastomers such as natural latex rubber, Kraton® rubbers such as styrene-butadiene and styrene-isoprene, nitrile rubber, polyurethane, neoprene, polybutadiene, polyisobutylene; thermoplastics and thermosets, such as polypropylene, polyethylene, ultra-high molecular weight polyethylene (UHMWPE), polytetrafluoroethylene (PTFE - Teflon), polyvinyldifluoride (PVDF), polyamide (Nylon), polyaramid (Kevlar), acetal plastic (Delrin), polystyrene, polyester, bakelite; and reinforced composites including, for example, any of the foregoing along with reinforcing materials such as glass fiber, carbon fiber, cellulose, hemp, and any other reinforcing material such as may be known in the art.

According to particular embodiments of the invention, the tires 122 of the caster wheels 116 may be pneumatic, semi-pneumatic, or solid. In still further embodiments, the caster wheels 116 may be untired, and may have circumferential surfaces of metal, or polymer coated metal, such as, for example, epoxy-coated steel.

It should be noted, however, that the bearing device may include no caster wheels at all, but may include an air bearing formed by actively pumping or releasing compressed air through channels and/or apertures in a lower surface of the base and adapted to reduce friction between lower surface of the base and the floor. Other non-caster bearing devices include, according to respective embodiments of the invention, polymer glides formed of, for example reinforced PTFE or of UHMWPE.

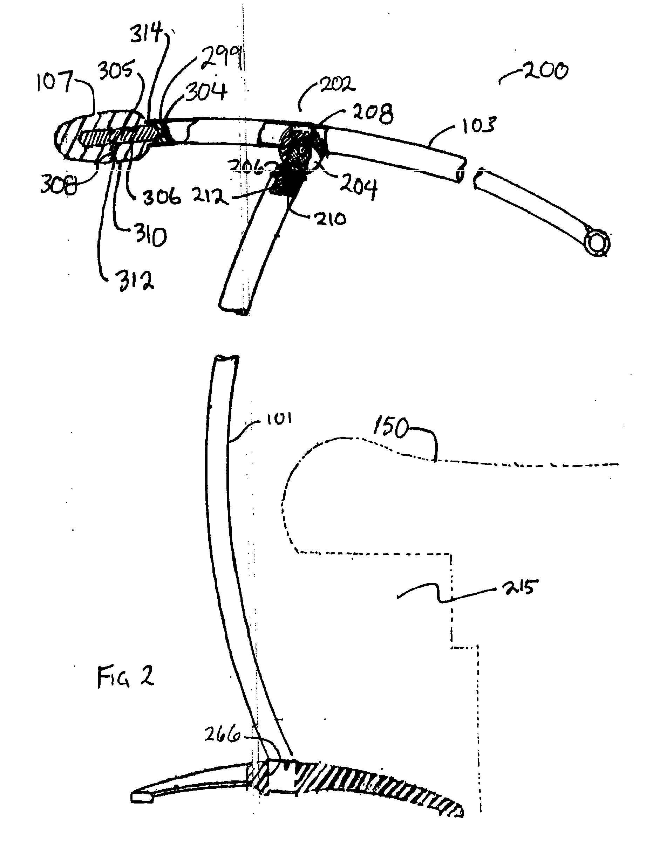

FIG. 2 shows, in sectional elevation, an apparatus support 200 for dentistry applications according to one embodiment of the invention. The FIG. 2 illustration presents, in some detail, the components of articulated member 104 of FIG. 1.

In one illustrated aspect, the apparatus support 200 includes a boom having a formation, for example, a boom joint such as a pivotal boom hinge 202 having at least two degrees of freedom. Specifically, in the illustrated embodiment, the boom hinge 202 has a first degree of freedom including rotational motion about a substantially vertical axis, and a second degree of freedom including rotational motion about a substantially horizontal axis. In the embodiment shown, rotation about the substantially horizontal axis may be effected by including within the boom hinge 202 at least one formation, which may include at least one or all of the following, for example, a boom joint yoke 204 and boom joint pivot 206 assembly having a hinge shaft 208 disposed between the boom joint yoke 204 and boom joint pivot 206.

Rotation about the substantially vertical axis is effected by including within the boom hinge 202 a boom pivot shaft 210 disposed within a substantially vertical bore of a boom top plug 212. Exemplary embodiments and aspects of boom hinges according to the invention are discussed in additional detail below.

As discussed above, in relation to FIG. 1, the articulated support member includes a mast 101 and a boom 103. In the illustrated embodiment, as is visible in FIG. 2, both the mast and the boom are curved.

According to a further embodiment of the invention, the curve of the boom 103 is a substantially circular curve. According to another embodiment of the invention, the curve of a boom may include a non-circular curve such as, for example, an elliptical curve, an ovoid curve, and a non-monotonic curve such as an "S" curve.

One advantage of the illustrated curvature of the mast and boom is that it optimizes the use of floor space in what may be an otherwise crowded dental examining room. For example, the curvature of the mast 101 may allow a larger portion of the base 102 to be received within a recess 215 under, for example a dental examining chair 150, table, or another piece of dental examining room equipment. In this way, otherwise usable space outwardly of the recess 215 is conserved. In another aspect, the curvature of the mast 101 and boom 103 may be aesthetically pleasing, and therefore contribute to the décor of the dental office. Nevertheless, the mast, and/or the boom may be substantially straight according to particular embodiments of the invention.

According to one embodiment of the invention, both the mast and the boom may be adapted to support signal devices and/or mass transfer devices. Exemplary signal devices may include filamentary signal carriers such as some metallic, ceramic, or conductive polymer wires, or optical fibers. In addition exemplary signal devices may include non-filamentary signal carriers such as radiofrequency waveguides. Exemplary mass transport devices include, for example, flexible polymer or metallic tubing adapted to the transportation of pressurized air or water.

In the illustrated embodiment, the mast and boom are also adapted to support power wiring for the dental instrument or apparatus.

According to one embodiment, the mast may include a concave side and a convex side, defining a curve in a plane perpendicular to the floor. At least one power pack having at least one formation, such as a mounting cable, may be attached to a formation of the mast, for example, a channel, on a convex side of the curved mast. As illustrated, the power pack includes a controller for controlling the lamp system.

FIG. 3a shows a rear elevation view of an exemplary mast 101 according to one embodiment of the invention. As shown, the mast includes a wire channel 152 disposed longitudinally in an external surface of the mast. In the illustrated embodiment, this wire channel 152 may extend from a lower end of the mast 101 to an upper end of the mast 101.

FIG. 3b shows, in cross-section, a mast 101 according to one embodiment of the invention. In FIG. 3b a profile of the wire channel 152 is visible, showing that it includes a recessed cavity disposed inwardly of an otherwise substantially elliptically cylindrical outer surface 154 of the mast 101. The wire channel 152 may include first and second lips 156. The first and second lips 156, according to the illustrated embodiment, may extend substantially along the length of the wire channel 152 from the lower end to the upper end of the mast 101. According to other embodiments, however, the lips 156 may be indented, to form intermittent projections along the length of the wire channel 152.

According to one embodiment of the invention, the mast may be formed of a metal or metallic alloy, such as stainless steel, extruded aluminum, an alloy such as Ni/Ti alloy; any amorphous metals including those available from Liquid Metal, Inc. or similar ones, such as those described in U.S. Patent No. 6,682,611, and U.S. Patent Application No. 2004/0121283, the entire contents of which are incorporated herein by reference.

According to another embodiment of the invention, the mast may be formed of any polymeric material. Suitable polymers include polyethylene, polypropylene, polybutylene, polystyrene, polyester, acrylic polymers, polyvinylchloride, polyamide, or polyetherimide like ULTEM®; a polymeric alloy such as Xenoy® resin, which is a composite of polycarbonate and polybutyleneterephthalate or Lexan® plastic, which is a copolymer of polycarbonate and isophthalate terephthalate resorcinol resin (all available from GE Plastics), liquid crystal polymers, such as an aromatic polyester or an aromatic polyester amide containing, as a constituent, at least one compound selected from the group consisting of an aromatic hydroxycarboxylic acid (such as hydroxybenzoate (rigid monomer), hydroxynaphthoate (flexible monomer), an aromatic hydroxyamine and an aromatic diamine, (exemplified in U.S. Patent Nos. 6,242,063, 6,274,242, 6,643,552 and 6,797,198, the contents of which are incorporated herein by reference), polyesterimide anhydrides with terminal anhydride group or lateral anhydrides (exemplified in U.S. Patent No. 6,730,377, the content of which is incorporated herein by reference)or combinations thereof.

In addition, any polymeric composite such as engineering prepregs or composites, which are polymers filled with pigments, carbon particles, silica, glass fibers, conductive particles such as metal particles or conductive polymers, or mixtures thereof may also be used. For example, a blend of polycarbonate and ABS (Acrylonitrile Butadiene Styrene) may be used for the lamp housing and head.

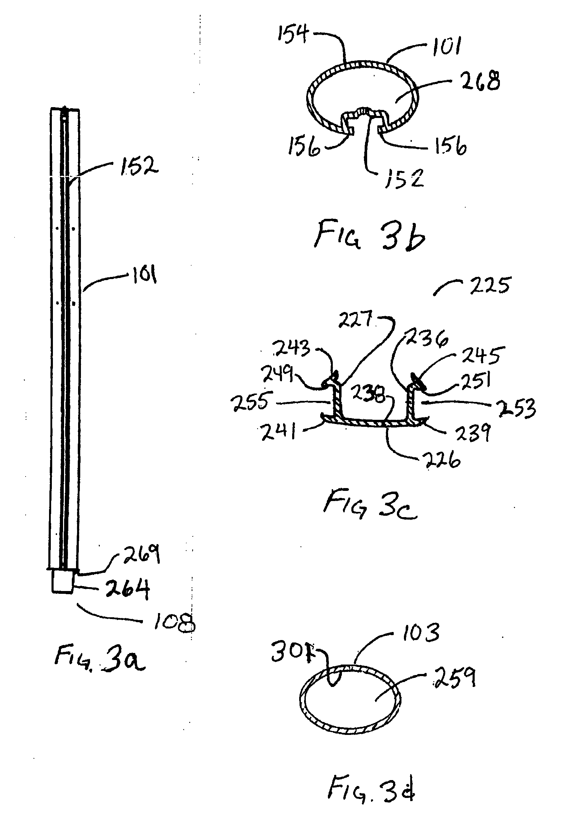

FIG. 3c shows, in cross section, a channel cover 225 according to one embodiment of the invention. The channel cover of FIG. 3c includes a plate member 226 and first and second projections 227, 236. The first and second projections 227, 236 are disposed substantially perpendicular to a rear surface 238 of the plate member 226. In addition, first and second projections are disposed inwardly of longitudinal edges 239, 241 of the plate member 226. Each projection, 227, 236, includes an angled barb 243, 245 with a respective back surface 249, 251. Consequently, each side of the channel cover 225 includes a respective longitudinal recess 253, 255 adapted to be mechanically coupled to a respective one of the lips 156 of the wire channel 152. This mechanical coupling may result in retention of the channel cover 225 adjacent to the wire channel 152 and effects closure of the wire channel 152.

According to one embodiment of the invention, the channel cover 225 is relatively inflexible, and the closure of the wire channel 152 is substantially permanent. According to another embodiment of the invention, the channel cover 225 is relatively flexible, and the channel cover 225 is therefore easily removable and replaceable subsequent to an initial installation.

In one embodiment of the invention, the channel cover 225 may be formed of a material including an elastomer such as those mentioned above.

According to another embodiment of the invention, the channel cover 225 may be formed of a metallic substance such as, for example, aluminum, steel, stainless steel, or those materials mentioned above in connection with the construction of the mast. According to still other embodiments of the invention, combinations of the foregoing materials, or of other materials, along with or exclusive of the foregoing materials, may be used according to the requirements of a particular embodiment.

In another embodiment of the invention, the boom 103 (as shown in FIG. 2) may also include a wire channel in a manner similar to the embodiment illustrated in FIG. 3b with respect to the mast 101. In such a case, a channel cover 225 like that of FIG. 3c may also be employed to cover the wire channel of the boom 103.

FIG. 3d shows, in cross-section, a boom 103 according to one embodiment of the invention. In the illustrated embodiment of FIG. 3d, the boom 103 does not include a wire channel. Instead, the boom 103 is substantially hollow, having an axial cavity 259 disposed therewithin. According to one embodiment of the invention, wires and/or tubing, for example, may be disposed and supported within the axial cavity 259.

The boom may be formed of one or more of the same materials as mentioned above for the construction of the mast, or of a different material. According to one embodiment of the invention, the boom may be formed of extruded aluminum.

Referring again to FIG. 3a, in accordance with one embodiment of the invention, the mast 101 includes a formation or coupling feature 108. The coupling feature 108 may be adapted to couple the mast 101, at its lower end, to a base 106 (as shown, for example, in FIG. 2). According to one illustrated embodiment of the invention, the coupling feature 108 includes a plug 264. In the illustrated embodiment, the plug 264 includes a substantially cylindrical outer surface, and may be adapted to be received within a formation in the base 106, such as a cavity having a substantially cylindrical inner surface 266 (as shown, for example, in FIG. 2).

According to one embodiment of the invention, the plug 264 may be substantially solid and formed, for example, as an integral casting. According to another embodiment of the invention, the plug 264 may be formed as an assembly of components.

According to still another embodiment of the invention, the coupling feature may include a projecting portion (not shown). The projecting portion may be adapted to be received within the interior cavity 268 of the mast 101. The projecting portion may be retained within the interior cavity 268 by, for example, a frictional force fit or by one or more threaded fasteners, or by spring pins, or other fastening means, according to various embodiments of the invention.

In a further aspect of the invention, according to one embodiment, the coupling feature 108 may include an alignment device 269. In one embodiment of the invention, the alignment device 269 may be a dowel or pin, such as a steel machine pin. In the illustrated embodiment, the pin may be a substantially cylindrical steel pin disposed within a bore and aligned substantially perpendicular to a longitudinal axis of the plug 264. In other embodiments (not illustrated here) the alignment device 269 may be a pin of rectangular cross section, a rectangular key, Woodruff key, roll pin, or other alignment device such as is known to one of skill in the art.

In one aspect of the invention, the alignment device 269 serves to maintain the mast 101 in a particular orientation with respect to the base 102 (as shown in FIG. 1). According to one embodiment, this orientation maintains the plane of curvature of the mast aligned with one of the beams 110 of the base.

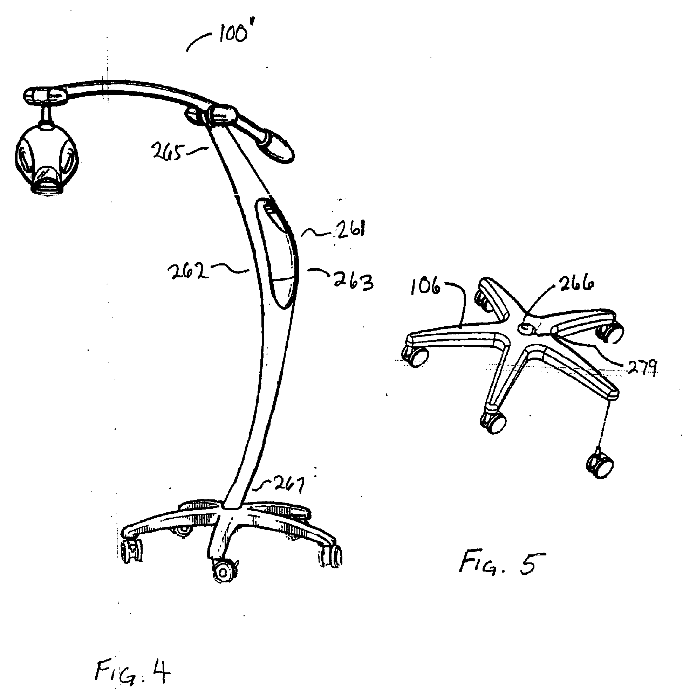

In one embodiment, the mast 101 may have a uniform outer dimension along its length, as shown in FIG. 1. In another embodiment, the mast 101 may have a non-uniform outer dimension along its length, as shown in FIG. 4.

FIG. 4 shows a dental equipment support structure according to another embodiment of the invention. As is apparent from the illustration of FIG. 4, the configuration of the mast, and also the boom, need not be strictly tubular. For example, the support structure 100' of FIG. 4 includes a mast 262 that has sides which diverge and subsequently converge with respect to one another as a function of height along the mast. Consequently, as shown in the illustrated embodiment, a cross-sectional area of the mast in a central region 263 is larger than the respective cross sections of the mast at an upper 265 region and a lower region 267.

In FIG. 4, the mid-section of the mast 101 is of a larger dimension than other parts of the mast. In one aspect, this mid-section may coincide with the mounting position of the power pack 261. In another aspect, the wider portion of the mast 101 may be flattened to accommodate a power pack 261. In a third aspect, the wider portion may be sunken or recessed to accommodate a power pack 261 so that the power pack 261 does not protrude far from the general profile of the mast 101.

In one embodiment of the invention, the boom 103 and mast 101 may be positioned such that their footprint does not exceed the footprint of the base 106. Specifically, when the boom 103 is rotated to a minimally vertical angle, whereby the lamp head is at its lowest elevation in proximity to the base, a projection of the lamp system on the floor falls entirely within the circumference of the base 106.

In another embodiment, the boom 103 and mast 101 may be positioned such that their footprint exceeds the footprint of the base 106 with the center of gravity of the dental lamp system falling within the base 106.

In an alternative embodiment of the invention, the outward-most surface of the counterweight 107 does not extend beyond the circumference of the base 107 in any angular position of the boom 103.

In one aspect of the invention, the mast 262 in FIG. 4 includes a cavity, or hollow region, within the central region 263. According to one embodiment of the invention, this hollow region is adapted to receive equipment such as, for example, a control module or power pack therewithin. Thus, according to one aspect of the invention, a streamlined and integrated support structure results. Such a support structure is advantageous in a dental office where projecting edges or equipment can snag clothing or otherwise interfere with professional activities.

FIG. 5 shows a perspective view of a base 106 according to one embodiment of the invention. As shown, the base 106 includes a formation, for example, a cavity having a substantially cylindrical inner surface 266. As noted above, this cavity may be adapted to receive the coupling feature 108 therewithin. In FIG. 5, there is also visible a slot 279 that opens into the cavity within base 106. According to one embodiment of the invention, this slot may be adapted to receive the alignment device 269 that projects from the substantially cylindrical outer surface of the coupling feature 108. According to one embodiment of the invention, as illustrated, a single slot 279 is present in the base. In other embodiments, a plurality of slots may be provided to receive a corresponding plurality of alignment devices 269.

Referring again to FIG. 2, and to the embodiment of the invention of illustrated therein, a counterweight 107 is disposed at a posterior end of the boom 103. In the illustrated embodiment, the counterweight 107 includes an axial bore 306 disposed inwardly from an aperture in an anterior surface of the counterweight.

In the illustrated embodiment, the boom includes an internal bulkhead 299 fixedly coupled to an internal surface 301 of the boom (see FIG. 3d) by, for example, welding or by the use of rivets or threaded fasteners. In other embodiments of the invention, the internal bulkhead is integrally formed as part of an extruded or molded boom structure.

According to one embodiment of the invention, the internal bulkhead 299 includes an internally threaded bore 304 disposed laterally therethrough. In one embodiment, the internally threaded bore 304 is disposed substantially coaxially with a longitudinal axis of a local region of the boom 103.

In the embodiment of FIG. 2, a counterweight shaft 305 is mutually disposed within the bore 306 of the counterweight 107 and within the internally threaded bore 304 of bulkhead 299. As shown, a further internally threaded bore 308 is disposed within the counterweight 107. Internally threaded bore 308 is disposed between respective apertures at bore 306 and outer surface 310 of counterweight 107. The further internally threaded bore 308 is disposed substantially perpendicular to bore 306. According to one embodiment of the invention, an externally threaded setscrew 312 is disposed within bore 308, and is adapted to be advanced inwardly from the surface 310 of the counterweight 107 such that an inward end of the setscrew 312 is disposed against an external surface 314 of the counterweight shaft 305.

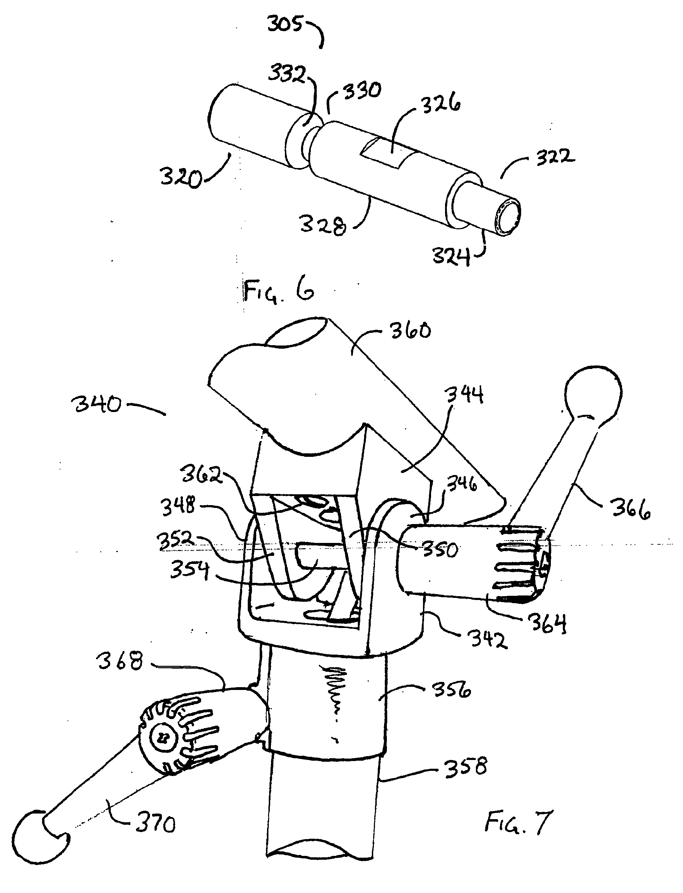

FIG. 6 shows the counterweight shaft 305 in additional detail according to one embodiment of the invention. As shown, the counterweight shaft 305 includes a posterior portion 320 adapted to be received within the axial bore 306 of the counterweight 107. The counterweight shaft 305 also includes an anterior portion 322 adapted to be received within the internally threaded bore 304 of bulkhead 299. In the illustrated embodiment, the anterior portion 322 includes a plurality of external threads 324. The plurality of external threads 324 is adapted to be threadingly coupled to a corresponding plurality of internal threads of the internally threaded bore 304 of the bulkhead 299.

In a further aspect, according to one embodiment of the invention, the counterweight shaft 305 includes at least one flat 326 disposed on an external surface 328 thereof. The flat 326 is adapted to receive a wrench, or other tool for the application of an axial torque about a longitudinal axis of the counterweight shaft 305. The application of this axial torque effects tightening, and therefore substantially fixed engagement, of the external threads 324 of the counterweight shaft and the internal threads of the internally threaded bore 304 of the bulkhead 299.

As would be understood by one of ordinary skill in the art, the fixed engagement of the external threads 324 of the counterweight shaft with the internally threaded bore of the bulkhead 299 may be further enhanced by the use of, for example, a lock washer, such as a split washer or a crown washer, or a thread locking solution as is known in the art.

In still another aspect, according to the FIG. 6 embodiment, the external surface 328 of the counterweight shaft 305 includes a circumferential groove 330. In one embodiment of the invention, circumferential groove 330 includes at least one side 332 that is disposed at an oblique angle with respect to a longitudinal axis of the counterweight shaft 305. According to one aspect of the invention, a corresponding oblique angle provided on a truncated conical inward end of setscrew 312 (as shown in FIG. 2) is effective to urge the counterweight into a fixed lateral position along a longitudinal axis of the counterweight shaft 305 with respect to, for example the bulkhead 299 as the setscrew 312 is advanced inwardly.

FIG. 7 shows, in perspective view, a boom joint 340 for a dentistry equipment support structure. The boom joint 340 includes a yoke 342 and a pivot 344. In the illustrated boom joint 340, the yoke includes first 346 and second 348 side members, having respective first and second bores disposed substantially horizontally therethrough. In like fashion, the pivot 344 includes third 350 and fourth 352 side members with respective third and fourth bores disposed substantially horizontally therethrough. The first, second, third and fourth bores are adapted to be aligned with an axle such as, for example, a carriage bolt 354 disposed coaxially therethrough. In this way, the yoke 342 and pivot to 344 are mutually supported in a pivoting relationship to one another by the axle 354.

The yoke 342 includes, for example, a female coupling feature 356. The female coupling feature 356 is adapted to be coupled to, for example, an upper end of a mast 358, such as the yoke is supported by the mast. As illustrated, the pivot 344 is coupled to a boom 360 by one or more bolts 362.

A first handle 364, has a first lever arm 366 and an internally threaded bore. The internally threaded bore may be adapted to receive an externally threaded end of the axle 354, whereby rotation of the handle serves to compress or release the side members 346, 348 of the yoke with respect to the corresponding sides 350, 352 of the pivot. A friction washer may be disposed between the side members and sides to modify the friction characteristics between side members and sides.

Compression of the side members 346, 348 serves to inhibit rotation about the axle 354 of the pivot 344 with respect to the yoke 342. Conversely, releasing the side members 346, 348 allows rotation about the axle 354 of the pivot 344 with respect to the yoke 342.

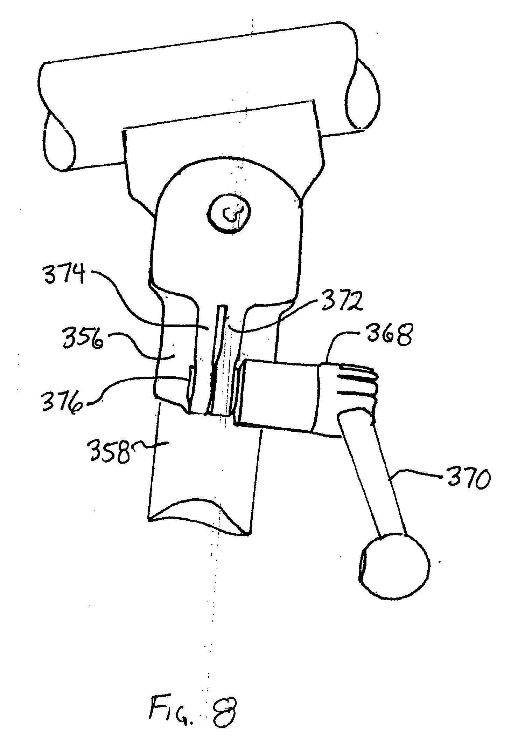

A second handle 368 has a second lever arm 370. Referring now to Fig. 8, one sees handle 368 in additional detail. As illustrated, the handle 368 includes an internally threaded bore adapted to receive an externally threaded bolt such as, for example, a carriage bolt 376. The female coupling 356 includes first 372 and second 374 flanges, each with a respective bore therethrough. The bores in flanges 372 and 374 are substantially aligned with one another, and adapted to receive the bolt 376. As will be clear to one of skill in the art, rotating handle 368 serves to compress and release the flanges 372, 374 with respect to one another. Compression of the flanges 372, 374 serves to inhibit rotation about the mast 358 of the yoke 342 with respect to the mast 358. Conversely, a releasing of flanges 372, 374 permits rotation of the yoke 342 with respect to the mast 358.

One of skill in the art will appreciate that operation of the boom joint of FIGs. 6 and 7 over an extended period of time will result in wear on one or more of the outer surface of mast 358, the corresponding formation, for example, the inner surface of female coupling feature 356, the outer surfaces of the third 350 and fourth side members 352 of the pivot 344 and the corresponding inner surfaces of the side members 346, 348 of the yoke 342. As such wear takes place, the frictional forces present at the respective interfaces changes accordingly. Consequently, the damping and resistance to motion response characteristics of the boom joint also change over the life of the support structure in ways that may be unpredictable and/or undesirable. The present invention aims to minimize such changes in such response characteristics. Other solutions are also possible, though some of them may be more, more difficult to produce, or more difficult to maintain in stable condition.

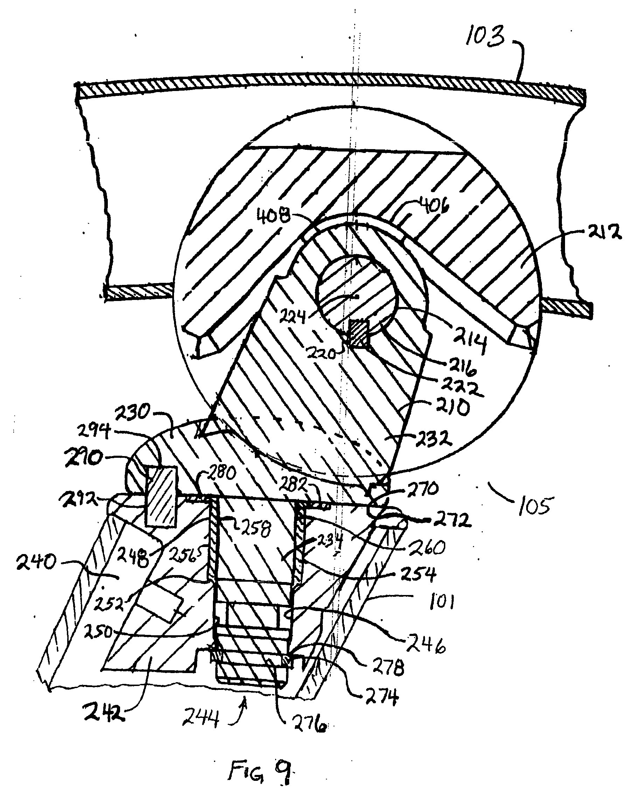

FIG. 9 shows, in cross-section, boom joint 105, according to one embodiment of the invention. As illustrated, the boom joint 105 includes a boom joint pivot 210, and a boom joint yoke 212. The boom joint pivot includes a horizontal bore 214 that is adapted to receive a boom joint shaft 216. The horizontal bore 214 is defined by an internal substantially cylindrical wall 218. According to the present embodiment, the wall 218 includes a slot 220. Referring again to FIG. 2, the slot 220 may be adapted to receive a portion of a fixturing device such as a Woodruff key 222.

The Woodruff key 222 aims to prevent rotation of the shaft 216 about an axis 224 that is common to both the shaft 216 and the bore 214. As will be seen more clearly in relation to further drawings discussed below, the yoke 212 may also include a bore that may be disposed about a portion of the shaft 216. In the embodiment of the invention presently under consideration, the yoke is not fixedly coupled to the Woodruff key 222 during use of the support structure. Consequently, the yoke 212, and the boom 108 that is fixedly coupled to the yoke 212, are rotatably supported by the shaft 216. The shaft 216 may be, in turn, supported by the pivot 210 and shaft 216.

The mast 101 and the boom 108 of the present invention may be made of any polymeric material, preferably a polymer that can be molded or cast; or a metal or metallic alloy. Suitable metal or metallic alloys, polymers and polymeric composite include those mentioned above

In the illustrated embodiment, the boom joint pivot 210 includes a base portion 230 as well as an upper portion 232. In various embodiments, the base portion 230 and the upper portion 232 ma be formed as a single integral unit, or as an assembly of separate components. In the illustrated embodiment, the base portion 230 may include a vertical shaft 234. Again, in various embodiments, the base portion 230 and vertical shaft 234 may include separate components, or they may form a single integral unit.

In one embodiment of the invention, the mast 101 may includes a substantially hollow bore or cavity 240. A mast top plug 242 may be disposed, fully or partially, within cavity 240. The mast top plug 242 may include an axial bore 244 defined by a substantially cylindrical internal surface 246. The radial diameter of the axial bore 244 may vary along the length of the bore such that a diameter of the bore is larger at a first location 248 than at a second location 250. Where the diameter of the bore 244 changes along the length of the bore, a ledge 252 exists.

According to one embodiment of the invention, a bushing 254 may be disposed within the axial bore 244. The bushing 254 may include a substantially cylindrical outer surface 256 disposed in substantially coaxial spaced relation to a substantially cylindrical inner surface 258.

In one exemplary embodiment, the bushing 254 may include and oil-filled porous bronze material. In another exemplary embodiment, the bushing 254 may include a polymer having a low coefficient of friction. This polymer may be selected from a variety materials including, but not limited to, for example, polyethylene, ultrahigh molecular weight polyethylene (UHMWPE), acetyl polymer materials and fiber reinforced acetyl polymer materials (Delrin), polyamide (Nylon), polyvinyl difluoride (PVDF), polytetrafluroethylene (Teflon), and other polymers of similarly desirable and appropriate characteristics, as well as those polymers, engineering prepregs and filled polymeric composites mentioned above for the construction of the mast and boom.

In another embodiment of the invention, the bushing 254 may include a material having a relatively high coefficient of friction, or an intermediate coefficient of friction, such that by interaction with outer surface 260 and inner surface 248, the bushing serves to damp and otherwise restrain a rotary motion of the vertical shaft 234 about its longitudinal axis.

As shown, a substantially cylindrical outer surface 260 of the vertical shaft 234 of the boom joint pivot 210 bears on, and is supported by, the substantially cylindrical inner surface 258 of the bushing 254. In addition, a substantially flat portion of a lower surface 270 of the base portion 230 of the boom joint pivot 210 bears on, and is supported by a substantially flat portion of an upper surface 272 of mast top plug 242.

A retaining device 274, such as a split-ring (c-ring) retaining washer may be mutually disposed within a groove 276 of the vertical shaft 234 and against a further ledge 278 of the substantially cylindrical surface of the axial bore 244. The retaining device 274 retains the vertical shaft 234 within the bore 244, and prevents the boom joint pivot 210 from moving upwardly with respect to the mast top plug 242.

According to this arrangement, the mast top plug 242 supports and retains the boom joint pivot 210 while allowing the boom joint pivot 210 to rotate about a mutual axis of the mast top plug bore 244 and boom joint pivot shaft 234.

In the illustrated embodiment, this rotation may be damped by the frictional action of a friction washer 280 disposed within a substantially circular recess 282 of the upper surface 272 of the mast top plug 242. One of ordinary skill in the art will appreciate that an outer circumference of the friction washer 280 may have any one of a wide variety of shapes including, but not limited to, an elliptical shape, a rectangular shape, a square shape, a sinusoidal shape, a toothed shape, and other shapes adapted to reduce rotation of the friction washer with respect to the mast top plug 242.

In various embodiments, the friction washer is formed to include a corresponding variety of materials including, but not limited to polyurethane, polybutylene, latex rubber, or other rubber materials that can be either natural or synthetic rubber. Synthetic rubbers are preferably elastomeric materials and include various copolymers or block copolymers (Kratons®) available from Kraton Polymers such as styrene-butadiene rubber or styrene isoprene, EPDM (ethylene propylene diene monomer) rubber, nitrile (acrylonitrile butadiene) rubber and the like, and other elastomers, cork, wood, ceramic materials, and other materials, or in combination, that are appropriate to the function of the friction washer. In addition the friction washer may include fibrous materials such as, for example, ceramic fiber, glass fiber, or mineral fiber materials among others.

In one embodiment of the invention, the damping frictional forces exerted between the friction washer 280, or other friction element, and the mast top plug 242 and the boom joint pivot 210 are sufficient to allow rotation of the boom joint pivot 210 when a force of between about 2 pounds and 5 pounds is exerted laterally against, or in proximity to the supported dental equipment.

In a further aspect of the invention, according to the embodiment illustrated in FIG. 9, a pin 290, such as a substantially cylindrical pin, is disposed mutually within a bore 292 in the upper surface 272 of the mast top plug 242, and within an arcuate slot 294 in the lower surface 270 of the base portion 230 of the boom joint pivot 210.

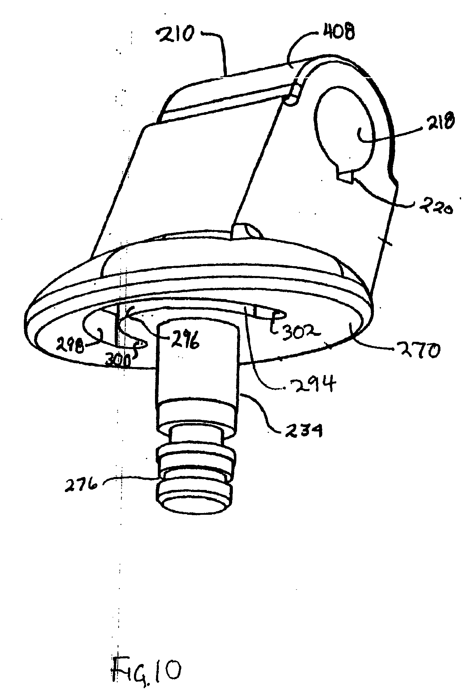

Turning now to FIG. 10 which shows the boom joint pivot 210 in a lower aspect of a perspective view, the arcuate slot 294 is more clearly visible. In the illustrated embodiment, the arcuate slot 294 includes a substantially vertical inner wall 296 and a substantially vertical outer wall 298. The arcuate slot 294 also includes substantially vertical end walls 300, 302. When viewed in relation to FIG. 9, it will be evident to one of ordinary skill in the art that when the boom joint pivot 210 may be assembled to the mast top plug 242, the boom joint pivot 210 will rotate freely to the extent that the pin 290 is disposed within the arcuate slot 294, away from the end walls 300, 302. When an outer surface of the pin 290 comes into contact with one or the other of the end walls 300, 302, rotation of the boom joint pivot, in a corresponding direction, is arrested.

FIG. 10 also shows a lower surface 270 of the boom joint pivot 210 that is supported by the upper surface 272 of the mast top plug 242. In addition, FIG. 10 shows the location of the slot 294 and the inner wall 296. One of ordinary skill in the art will understand that alternative locations for the slot 294 will also serve. For example, in one embodiment of the invention, an arcuate slot similar to slot 294 may be provided in upper surface 272 of mast top plug 242 (as shown in FIG. 9). Correspondingly, a bore similar to that shown 292 in mast top plug 242 is provided in lower surface 270 of boom joint pivot 210 with a pin 290 disposed therein.

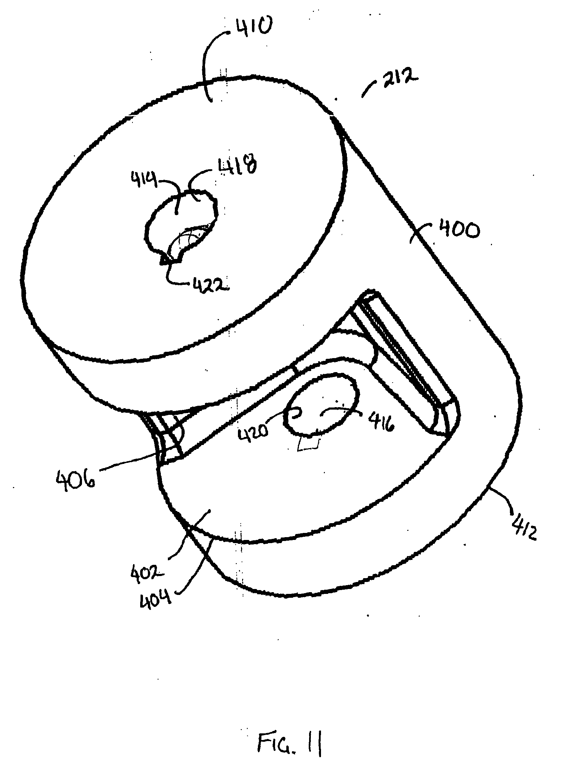

FIG. 11 shows the boom joint yoke 212 in a ventral aspect of a perspective view according to one embodiment of the invention. As shown, the yoke 212 has a substantially cylindrical outer surface 400. An aperture 402 in the outer surface is defined by an edge 404 shaped substantially as a projection of a rectangle on cylindrical surface 400.

The aperture 402 opens upon an internal cavity within the yoke 212. Referring again to FIGs. 9 and 10, one sees that a rear wall 406 of the cavity 402 is configured to be disposed in spaced relation to an outer surface 408 of boom joint pivot 210.

As shown in FIG. 11, the yoke 212 also includes first 410 and second 412 (not visible) substantially circular end surfaces. According to one embodiment of the invention, the end surfaces 410 and 412 are substantially flat. Each end surface 410, 412 has a respective bore 414, 416 disposed coaxially through the surface and normal thereto.

Each bore 414, 416 is defined by a respective substantially cylindrical wall 418, 420. As is discussed above, in relation to FIG. 9, walls 418 and 420 serve to rotatably support the yoke 212 on shaft 216.

It is noted that, while wall 418 is shown to have a keyway slot 422, and while according to various embodiments, both wall 418 and wall 420 include such a keyway slot, the keyway slot is merely to allow the Woodruff key 222 (as shown in FIG. 9) to pass through yoke 212 and into slot 220 of boom joint pivot 210. Accordingly, shaft 216 is rotationally fixed with respect to boom joint pivot 210, but is rotatable with respect to the yoke 212.

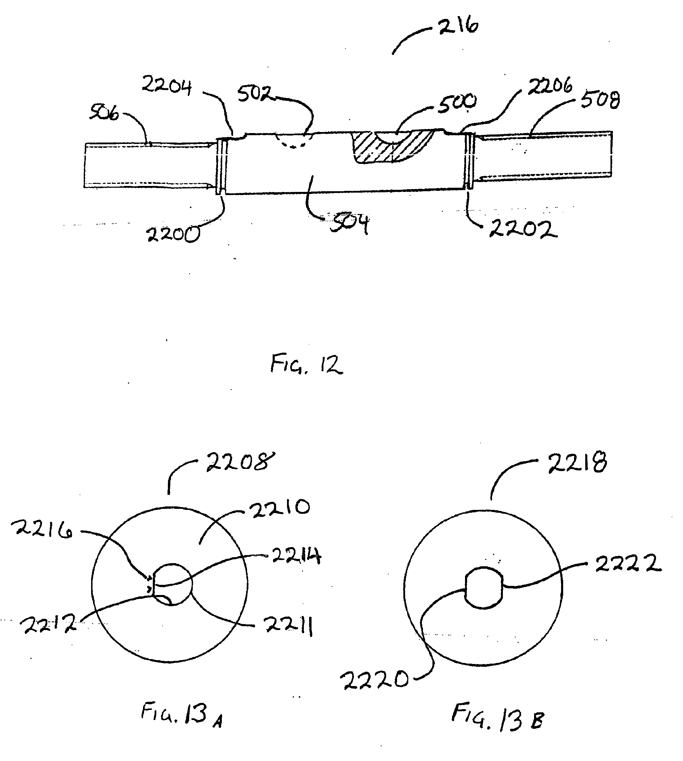

FIG. 12 shows the boom joint shaft 216 in a cutaway view. In the illustrated embodiment, the shaft may include two cavities 500, 502 adapted to receive two respective Woodruff keys. In another embodiment, the shaft may include a single Woodruff key cavity and only a single Woodruff key is employed. In still another embodiment of the invention, a slot, adapted to receive a machine screw, runs all or part of the length of the surface 504 of the boom joint shaft 216. One of skill in the art will understand that the use of machine keys or Woodruff keys is merely exemplary of the many ways in which the boom joint shaft 216 may be maintained rotationally fixed with respect to the boom joint pivot 210.

Also shown are external threads 506, 508 on the outer surface of the boom joint shaft 216. These threads are adapted to receive knobs 109 and 111 (as shown in FIG. 1) respectively. According to one embodiment of the invention, the threads 506, 508 are configured with a spacing of, for example, about 20 threads per inch, however any number of standard or non-standard thread sizes may be employed as appropriate.

The shaft 504 of FIG. 12 also may include first 2200 and second 2202 circumferential grooves as illustrated. The circumferential grooves 2200, 2202 may be adapted to receive respective retaining devices therewithin. For example, each groove 2200, 2202 may be adapted to hold a snap ring retainer.

In addition, shaft 504 includes first 2204 and second 2206 flat regions. As will be explained in additional detail below the flat regions 2204, 2206 are adapted tominimize or prevent rotation of boom joint washers with respect to the shaft 504.

FIG. 13a shows a boom joint washer 2208 according to one embodiment of the invention. The boom joint washer 2208 may include an outer surface 2210 and an inner surface (not shown). A hole through the washer may be bordered by an edge having a first substantially circular portion 2212 and a second substantially flat portion 2214. In one embodiment of the invention, a profile of the substantially flat portion 2214 may be adjusted by a depression 2216 in the outer surface of the washer. In the illustrated example, this depression may be formed by the application of a punch to the outer 2210 surface. By thus adjusting the profile of the substantially flat portion, a tight fit to the shaft 504 having minimal play may be achieved.

FIG. 13b shows another boom joint washer 2218 according to another embodiment of the invention. The boom joint washer 2218 includes a hole having an edge with two substantially flat portions 2220, 2222. As will be discussed below, the use of two flats and improved manufacturing tolerances obviates the need to adjust a profile of the flat portions 2220, 2222 according to one embodiment of the invention.

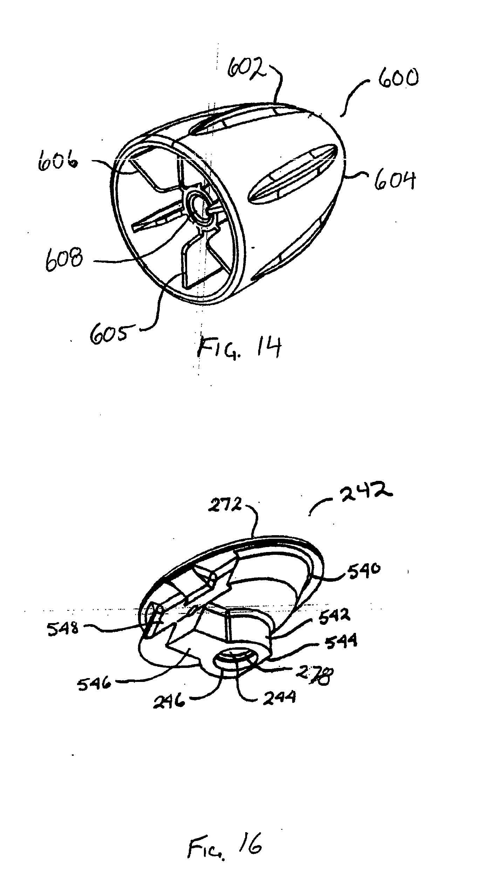

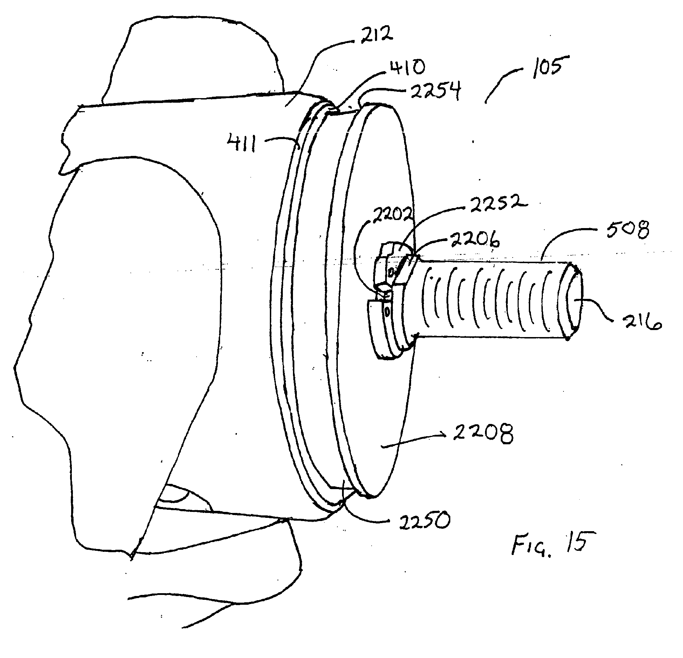

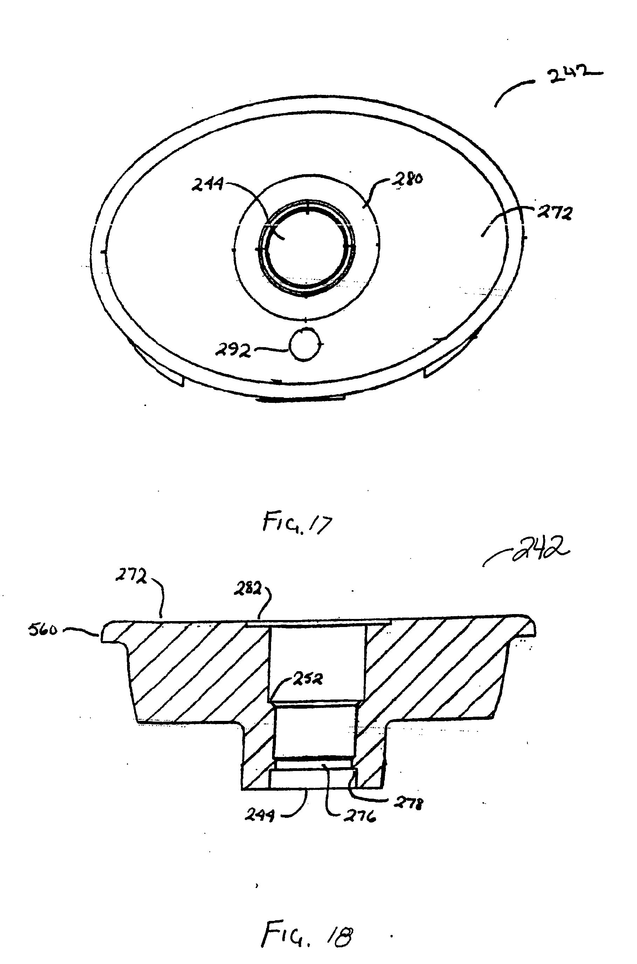

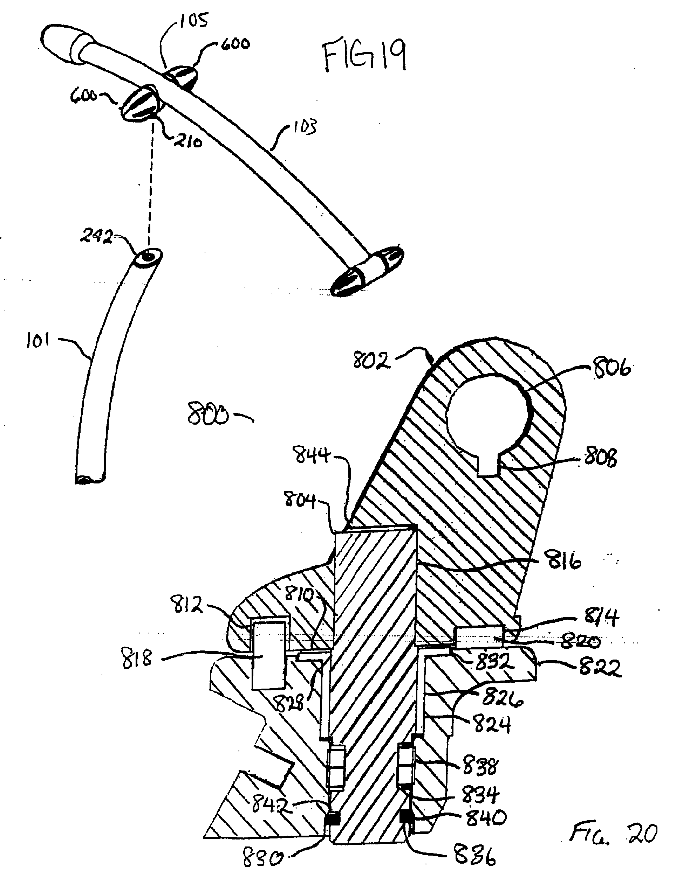

FIG. 14 shows a perspective view of a boom hinge knob 600 according to one embodiment of the invention. According to the illustrated embodiment, the boom hinge knob 600 may include a substantially hemi-ellipsoid outer surface 604. In the embodiment shown, a plurality of projections 602 may be disposed around the outer surface 604 of the boom hinge knob 600. The projections 602 provide a gripping surface for a user of the supported dental equipment, making it easy for the user to turn the knob 600 in order to make adjustments to the boom joint 105. In an alternative embodiment of the boom hinge knob 600, the outer surface 604 is fluted instead which also provides a gripping surface for the dental whitening lamp user. In a further embodiment, a plurality of raised or depressed striations can also be provided on the gripping surface to facilitate gripping.

A plurality of ribs 606 on the inside of the boom hinge knob 600 provide structural reinforcement for the knob 600 and also support a cylinder 608 with internal threads. Each rib 606 may include an outwardly facing surface 606. As will be described in additional detail below, the outwardly facing surface may be adapted to contact the surface (e.g., 2210) of the boom joint washer. The threaded cylinder 608 may be centered inside the boom hinge knob 600 and may be shaped and configured to receive the threaded end 508 of the boom joint shaft 216. This arrangement enables adjustment of the boom joint 105.

According to one embodiment of the invention, the internally threaded cylinder 608 may be coupled to the knob 600 by a frictional press fit. In another embodiment of the invention, the internally threaded cylinder 608 may be coupled to the knob 600 by means of ultrasonic welding during an assembly operation. In still another embodiment of the invention, the knob 600 may be formed of a durable material capable of supporting threads formed directly in an internal surface of the knob.

FIG. 15 shows in perspective view, a portion of a boom joint according to one embodiment of the invention. The boom joint 105 includes a yoke 212 having a substantially flat surface 410 with a substantially circular perimeter 411. Also shown is a friction washer 2250 with a substantially circular perimeter and a boom joint washer 2208 as discussed above in relation to FIG. 13a. A boom joint shaft 216 is shown supporting the yoke 212, friction washer 2250 and boom joint washer 2208. In addition a split ring retainer 2252 is shown disposed within a groove 2202 of the boom joint shaft 216.

The boom joint shaft 216 may include a plurality of external threads 508. The external threads are adapted to receive a boom joint knob 600 such as that described above in relation to FIG. 14.

One of skill in the art will appreciate that the presence of respective retaining devices e.g., 2252 in grooves 2200 and 2202 of the shaft 504 (as shown in FIG. 12) substantially prevents axial motion of the shaft 506 with respect to the yoke 212. Also, rotation of the shaft 506 about its long axis is substantially prevented by the presence of a woodruff key (or other device) coupling the shaft 506 to the boom joint pivot 210.

Rotation of the boom joint washer 2208 with respect to the shaft 216 is prevented by the action of flat surface 2206 of shaft 216 on flat surface 2212 of the joint washer 2208.

Rotation of the boom joint knob 600 may cause the knob to advance axially inwardly with respect to shaft 216. Consequently surfaces 605 of the boom joint knob may impinge on surface 2210 of the boom joint washer 2208. The washer 2208, may thus be urged axially inwardly to compress the friction washer 2250 between the inwardly facing surface 2254 of the boom joint washer and an adjacent face of the friction washer. The friction washer may correspondingly be urged against the surface 410 of the yoke 212.

Friction between the friction washer 2250, surface 410 of the yoke 212 and surface 2254 of the boom joint washer tends to couple the yoke to the boom joint washer. A chain of mechanical coupling is thus established from the boom 103 and yoke 212, which are substantially fixedly coupled to one another, through the friction washer 2250 to the boom joint washer 2208, and, by way of the flat surface 2212 of the washer 2208 and the flat surface 2206 of the shaft 216, through the shaft and woodruff key 222 to the boom joint pivot 210, and thus to the mast 101 which supports the boom joint pivot. In view of the foregoing, one of skill in the art will appreciate that the resistance to relative motion between mast 101 and boom 103 is adjustable by rotation of the boom joint knob 600.

FIG. 16 shows a ventral aspect, in perspective view, of a mast top plug 242. As described above, the mast top plug 242 is adapted to be disposed within a hollow bore 240 of the mast 101. The mast top plug 242 has a generally cylindrically-shaped underside 540 that tapers down to a key-shaped portion 542 that has a first flat 544 and a protrusion. A second flat 548 is located above the protrusion under the upper surface 550 of the mast top plug 242.

In the present embodiment of the mast top plug 242, the mast top plug 242 may be shaped and configured to fit into the hollow bore 240 of the mast 101 in one orientation. The first flat 544, second flat 548 and protrusion 546 may determine an orientation of insertion into the hollow bore 240 of the mast 101.

The mast top plug 242 may include an axial bore 244 defined by a substantially cylindrical internal surface 246. The axial bore 244 receives the shaft 234 of the boom joint pivot 210. The axial bore 244 may include a ledge 278 in the substantially cylindrical surface 246. The retaining device 274 (described in FIG. 9) around the shaft 234 of the boom joint pivot 210 may rest against the ledge 278. As described above with regard to FIG. 2, the retaining device 274 may retain the shaft 234 within the axial bore 244. Accordingly, the mast top plug 242 supports and retains the boom joint pivot 210 while allowing the boom joint pivot 210 to rotate about a mutual axis of the bore 244 and vertical shaft 234.

In one embodiment of the mast top plug 242, the mast top plug 242 may fit snugly to the mast 101. In an alternative embodiment of the mast top plug 242, the upper edge of the mast top plug 242 may have a groove 552 holding an O-ring. The O-ring 554 provides a tight fit in the hollow bore 240 of the mast 101.

FIG. 17 shows a top view of mast top plug 242. The top of the mast top plug 242 may be oval-shaped in the present embodiment of the invention to match the oval configuration of the mast 101. Other general shapes are contemplated within the scope of the invention. The mast 101 and mast top plug 242 are not limited to the shape shown in FIG. 17.

The upper surface 272 of the mast top plug 242 is substantially flat and, as described above, supports the substantially flat surface 270 of base portion 230 of the boom joint pivot 210. According to one embodiment, a friction washer 280 is disposed within a recess 282 in the upper surface 22(seen in FIG. 10). The friction washer 280 damps the rotation of the boom joint pivot 210 in relation to the mast top plug 242. As described above, in one embodiment of the invention, the damping frictional forces exerted between the friction washer 280 and the mast top plug 242 and the boom joint pivot 210 are sufficient to allow rotation of the boom joint pivot 210 only when a force of between about 2 pounds and 5 pounds is exerted laterally against, or in proximity to the payload apparatus.

The mast top plug 242 may include an aperture opening to a bore 292 at the upper surface 272 and extending into the mast top plug 242. The bore 292 may be shaped and configured to receive a pin 290. As shown in FIG. 9, the pin 290 is disposed mutually within a bore 292 in the upper surface 272 of the mast top plug 242, and within an arcuate slot 294 in the lower surface 270 of the base portion 230 of the boom joint pivot 210.

FIG. 18 shows a cross-sectional view of the mast top plug 242, as taken along the long axis of the plug. The mast top plug 242 includes the axial bore 244 shaped so that there are two ledges 252, 278. Ledge 278 bears the retaining device 274 disposed in the groove 276 of the boom joint pivot 210 as seen in FIG. 2. The recess 282 in the upper surface 272 coaxial with the axial bore 244 may receive the friction washer 280 (shown in FIG. 17). In this embodiment, the mast top plug 242 includes a rim 560 that encircles an upper surface 272 of the mast top plug 242. A lower surface 561 of rim 560 rests on top of the mast 101. The rim 560 may be shaped and configured to match an upper surface of the mast 101 so that the mast top plug 242 fits flush to the mast 101.

FIG. 19 shows an assembly view of a mast 101, boom 103 and boom joint 105 according to one embodiment of the invention. The mast top plug 242 is disposed within the mast 101. The boom joint pivot 210 is shown assembled into the boom 103. A knob 600 on either side of the boom joint 105 serves to fix and release an angular orientation of the boom 103 to be adjusted in angle with respect to the mast 101.

FIG. 20 shows, in cross-section, a mast-top plug and pivot assembly 800 according to another embodiment of the invention. The pivot 802 includes a vertical bore 816 adapted to receive a pivot shaft 804. The pivot may further include a horizontal bore 806 that is adapted to receive a boom joint shaft 216 (not shown) in similar fashion to the embodiment of a boom joint 200 shown in FIG. 2. The bore 806 may include a slot 808 adapted to receive a portion of a fixturing device, again, in similar fashion to the embodiment of the boom joint 200 shown in FIG. 2. The pivot 802 has a substantially flat lower surface 810. The lower surface 810 may include a first arcuate slot 812 and a second arcuate slot 814 located in opposition around the vertical bore 816. The first arcuate slot 812 may be adapted to receive a first pin 818. The second arcuate slot 814 may be adapted to receive a second pin 820.

The first pin 818 and second pin 820 are mounted in a mast top plug 822. The mast top plug 822 includes an axial bore 824 defined by a substantially cylindrical internal surface 826. The radial diameter of the axial bore 824 varies along the length of the bore such that a diameter of the bore is larger at a first location 828 than at a second location 830. According to one embodiment of the invention, a bushing 832 is disposed within the axial bore 824. The bushing 832 may include a substantially cylindrical outer surface disposed in substantially coaxial spaced relation to the substantially cylindrical inner surface 826 of the axial bore 824. The bushing may be made from, for example, a material having a low coefficient of friction. The pivot shaft 804 is disposed within the bushing 832 within the axial bore 824. The pivot shaft 804 has a first groove 834 and a second groove 836. A friction ring 838 is disposed within the first groove 834. The friction ring 838 is, for example, made of rubber. In an alternative embodiment of the invention, there are two or more friction rings disposed within the first groove 834. A retaining device 840, such as a split-ring (c-ring) retaining washer is mutually disposed within the second groove 836 of the pivot shaft 804 and against a ledge 842 of the substantially cylindrical surface of the axial bore 244. The retaining device 840 retains the pivot shaft 804 within the bore 824, and prevents the pivot 802 from moving upwardly with respect to the mast top plug 822. In one embodiment of the invention, the pivot shaft 804 is welded to the pivot 802 at point 844. In another embodiment of the invention, the pivot shaft 804 is retained in the pivot 802 with a screw in a fashion similar to the embodiment described with regard to FIG. 18.

According to this arrangement, the mast top plug 822 supports and retains the pivot 802 while allowing the pivot 802 to rotate about a mutual axis of the mast top plug bore 824 and pivot shaft 804. The rotational movement in this embodiment is limited by the movement allowed by the first pin 818 and second pin 820 within the first arcuate slot 812 and second arcuate slot 814 respectively. The movement is, for example, limited to 180 degrees.

In the illustrated embodiment, the rotational movement is damped by the frictional action of the friction ring 838 disposed within the first groove 834 on the pivot shaft 804 in side the axial bore 824.

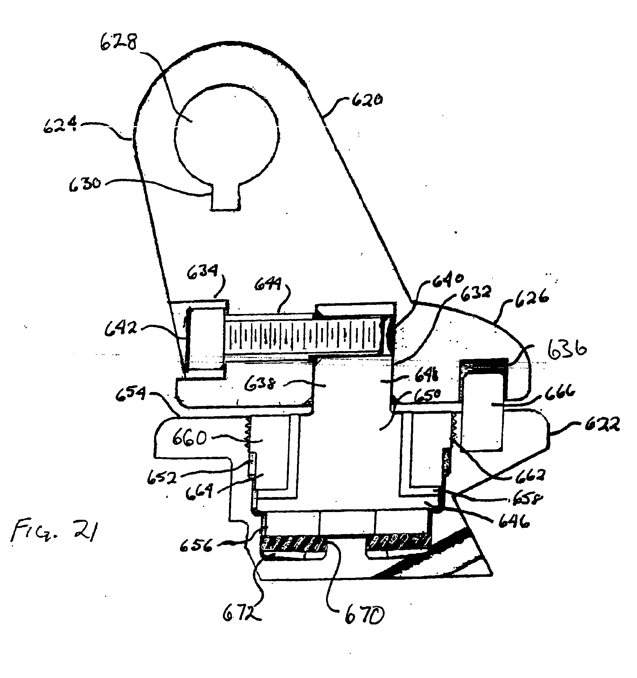

FIG. 21 shows, in cross section, a boom joint pivot 620 and mast top plug 622 for a boom joint according to another embodiment of the invention.

The boom joint pivot 620 may include an upper portion 624 and a base portion 626. The upper portion 624 of the boom joint pivot 620 includes a horizontal bore 628 that is adapted to receive the boom joint shaft 216 in similar fashion to the embodiment of a boom joint 105 shown in FIG. 30. The bore 628 includes a slot 630 adapted to receive a portion of a fixturing device, such as a woodruff key in similar fashion to the embodiment of the boom joint 105 shown in FIG. 9.

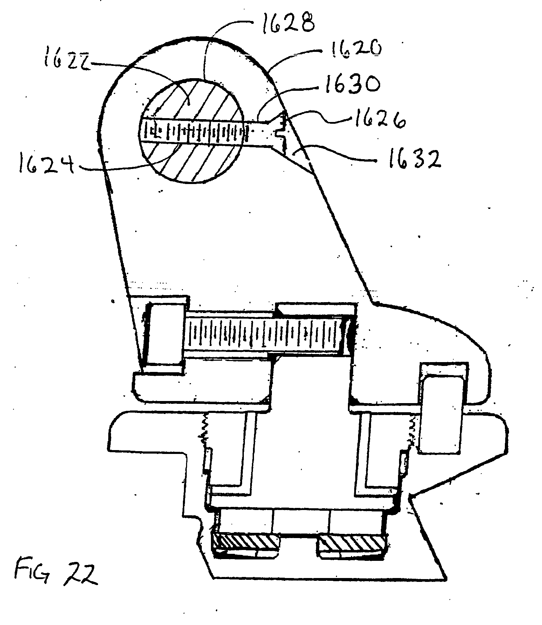

FIG. 22 shows, in cross section, a further embodiment of a boom joint pivot 1620 according to the invention. As shown in FIG. 20, the pivot 1620 includes a bore formed therethrough. The bore is adapted to receive a boom joint horizontal pivot shaft 1622. The shaft 1622 of the present embodiment does not include a woodruff keyway such as that identified as 502 in the boom joint pivot shaft 216 of FIG. 12. Rather, the shaft 1622 of the present embodiment includes a bore 1624 may be formed within the shaft 1622 and substantially perpendicular to a longitudinal axis of the shaft 1622. According to one embodiment of the invention, the bore 1624 includes an internal surface bearing threads or other feature adapted to retain a fastener within the bore 1624.

In the illustrated embodiment, the fastener is shown as a flat head machine screw 1626. One of skill in the art will appreciate, however, that a wide variety of other fasteners may be substituted for the machine screw 1626. For example, the fastener employed may include one or more of a roll pin, a headless hex screw (set screw) a cap screw, a rivet, and a dowel such as a steel dowel, among others.

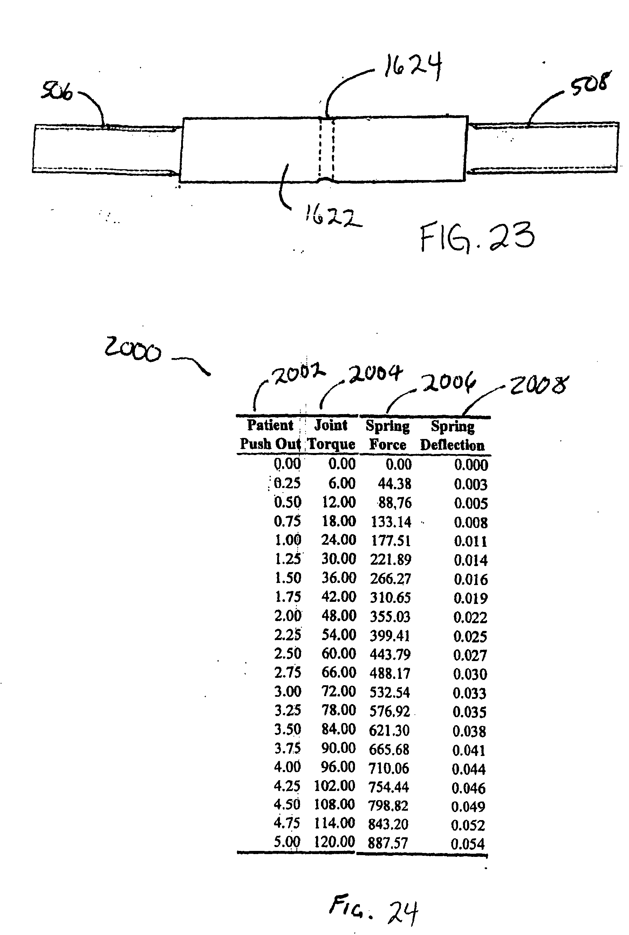

FIG. 23 shows the boom joint horizontal pivot shaft 1622 in additional detail. As shown, the shaft includes a through hole 1624 as well as externally threaded ends 506, 508. In various embodiments of the invention, the through hole 1624 may be internally threaded to receive a machine screw or smooth to receive a fastener such as a roll pin.

Referring again to FIG. 22, in one embodiment of the invention, an adhesive material may be disposed at an interface 1628 between an external surface of the shaft 1622 and an inwardly facing surface of the bore within which the shaft 1622 may be disposed. In various embodiments, the adhesive material may include a cyanoacrylate based material such as, for example Loc-Tite™ or Super Glue™, other structural bonding adhesives including an epoxy, one or two part, polyurethane adhesives, one or two parts, or a foam mounting adhesive. The foam mounting adhesive may also aid in shock absorption.

In another embodiment of the invention, the bore within the pivot 1620 that receives the screw 1626 or other fastener may include a recessed region such as, for example, a countersunk region 1632. The recessed region is adapted to receive a head of a fastener, so as to prevent interference between the head of the fastener and a yoke, such as that shown as 212 in FIG. 11. In still another embodiment of the invention, the bore 1630 traverses the shaft 1622 and extends into the pivot 1620 at the far side of the shaft. In a further embodiment of the invention, the bore 1630 extends completely through the pivot 1620.

The base portion 626 of FIG. 21 includes a vertical bore opening 632 and a horizontal bore opening 634. The base portion 626 further includes an arcuate slot 636, which can be seen more clearly in FIG. 29.

Referring again to FIG. 21, a vertical bore opening 632 may be adapted to receive a pivot shaft 638 having a through-hole 640. The horizontal bore opening 634 may be adapted to receive a screw 642. In a first embodiment of the invention, the horizontal bore opening 634 has a threaded portion 644 adapted to mate with threads on the screw 642. In an alternative embodiment, the through-hole 640 of the pivot shaft 638 may be threaded and mates with threads on the screw 642.

The pivot shaft 638 is generally cylindrical in shape and may include the through-hole 640 at one end and a flange 646 at the other end. In one embodiment of the invention, the pivot shaft 638 has a first portion 648 having a smaller diameter and a second portion 650 having a larger diameter.

The mast top plug 622 may include a generally cylindrical opening 652 axially located and extending from the upper surface 654 of the mast top plug 622. The opening 652 may configured to receive a friction pad 656. The friction pad 656 provides greater friction resistance with increasing compressive force against it. The friction pad 656 may for example, made of an elastomeric material. The opening 652 may further configured to receive the pivot shaft 638 where the flange 646 of the pivot shaft 638 contacts the friction pad 656. The opening 652 may partially threaded near the upper surface 654 of the mast top plug 622. A bushing 658 fits over the pivot shaft 638. The opening 652 may be still further configured to receive a spanner nut 660. The spanner nut 660 has an upper portion 662 that is threaded and a lower portion 664 that is not threaded. The lower portion 664 of the spanner nut contacts the bushing 658 while the threaded portion 662 of the spanner nut 660 screws into the opening 652.

In operation, the boom joint pivot 620 is fixedly joined to the pivot shaft 646 with the screw 642. The pivot shaft 646 rotates within the bushing 658 inside the mast top plug 622. The rotation of the pivot shaft 646 may be damped by the frictional action of the pivot shaft 646 against the friction pad 656. The force needed to rotate the pivot shaft 646 may be adjustable by adjusting the amount of compression of the pivot shaft 646 against the friction pad 656 provided by the spanner nut 660.

In one embodiment of the invention, the characteristics of the friction pad 656 may be selected to provide a desirable resistance to rotation of the boom with respect to the mast. Smooth and predictable motion of the boom with respect to the mast is of significant value in the dental practitioner's office. It turns out that a patient's ability to push away dental equipment, such as a whitening lamp head, leads, anecdotally, to surprisingly increased comfort levels for the patient during a dental procedure.

At the same time, it is advantageous that the motion of the boom be sufficiently damped so that the lamp head may remain in a substantially fixed position until its motion is desired by the patient or dental practitioner. For example, a boom hinge embodiment, as illustrated in FIG. 15 shows significantly improved characteristics as compared to other boom hinge arrangements such as, for example, that of FIG. 7 or that found in exemplary published conventional lamp support structures such as that of U.S. patent number 3,031,215 issued April 24, 1962 to Vance, or U.S. patent number 4,671,478 issued June 9,1987 to Schoenig et al., the disclosures of which are herewith incorporated by reference in their entirety.

Consequently, calculations as to the characteristics of the joint have been performed with respect to one exemplified embodiment. The calculations performed serve to characterize the forces applied to the friction washer 656 by the various components of the boom joint, and the expected performance of the assembly, including projected operative lifetime of the friction washer 656.

Anecdotal evidence suggests that patient push out force is a surprisingly important factor in overall patient comfort and effectiveness of a dental equipment support structure. The optimal support structure must provide sufficient resistance to displacement to effectively support equipment and, in some embodiments, maintain it substantially immobile with respect to the teeth of a patient. At the same time, the support structure may allow the patient to displace the equipment without unreasonable effort when desired. Also, in some embodiments, it is desirable to have sufficient mobility of the support structure to allow the supported equipment to accommodate minor and/or involuntary movements of the patient. Further, it is desirable that, subject to initial adjustment, the support structure be capable of adjustment by the application of forces near and/or on the supported equipment, such that there is no need for constant referral to portions of the structure that are relatively remote from the equipment, such as the boom joint or caster locks.

This combination of features and functions is surprisingly important to the overall perception of functionality and ease of use by the patient and dental practitioner and is not satisfied by the various conventional support mechanisms known to be available. Furthermore, achieving an optimal combination of characteristics has been found to surprisingly sensitive to aspects of the invention as embodied in particular design features.

For example, an embodiment of the invention including the boom joint of FIG. 19 is found to provide highly linear and stable load bearing characteristics that maintain consistency and repeatability over a large number of operative cycles. Thus it is found that the application of particular forces at the load (i.e., the equipment being supported by the equipment support structure) results in a load displacement that is surprisingly consistent and repeatable over a large number of operative cycles. In this way, the problem of maintaining effective support and control of an article of dental equipment, while allowing appropriate repositionability and patient push-out characteristics is solved by application of the present invention in its various aspects.

FIG. 24 shows a table of forces 2000. The table 2000 relates to the force applied at the load end of the boom to produce a responsive motion of the boom with respect to the mast. This applied force, identified as patient push out 2002, represents a force required to patient to, for example, push a whitening lamp head that may be coupled to the boom away from the patient's mouth.

The joint torque corresponding to a particular push out force is shown as 2004. Also shown are the spring force 2006 (in pounds) applied to the friction washer in one embodiment of the invention, and the corresponding spring deflection (in inches) 2008 for a particular Bellevue washer spring. Thus, for example, in the illustrated embodiment, a spring force of about 355 pounds may produce a spring deflection of about 0.022 inches. The resulting joint torque is 48 pounds which translates into a patient push out force of 2 pounds.

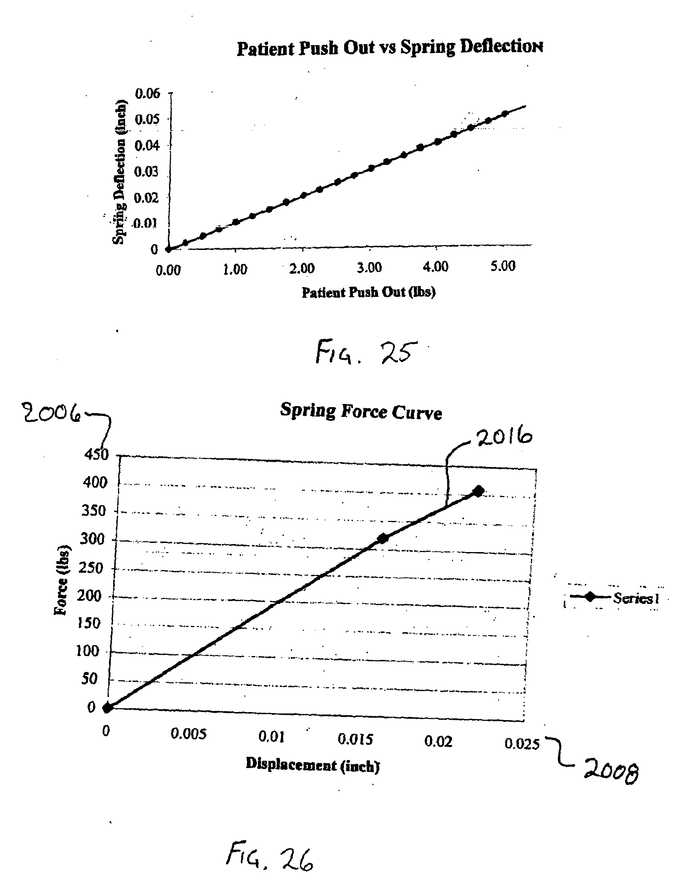

FIG. 25 shows a graphical representation of the relationship 2010 between spring deflection 2012 (in inches) and patient push out force (in pounds) 2014. In the illustrated embodiment, this relationship is substantially linear.

Figure 26 shows a graphical representation of a spring force function 2016 characteristic of a boom joint apparatus according to one embodiment of the invention. The vertical axis represents a force 2006 applied to the spring in a substantially axial direction. The horizontal axis represents a displacement 2008 in inches of the spring responsive to the applied force 2006. As shown in figure 26, the displacement of a spring, such as a Bellevue washer, according to the illustrated embodiment, is substantially proportional to the axial force 2006 applied to the spring. Accordingly, the graphical line representing the relationship 2016 is substantially linear. One of skill in the art will appreciate that springs, including integral and composite springs, having other spring force characteristics may be applied in various embodiments of the invention.

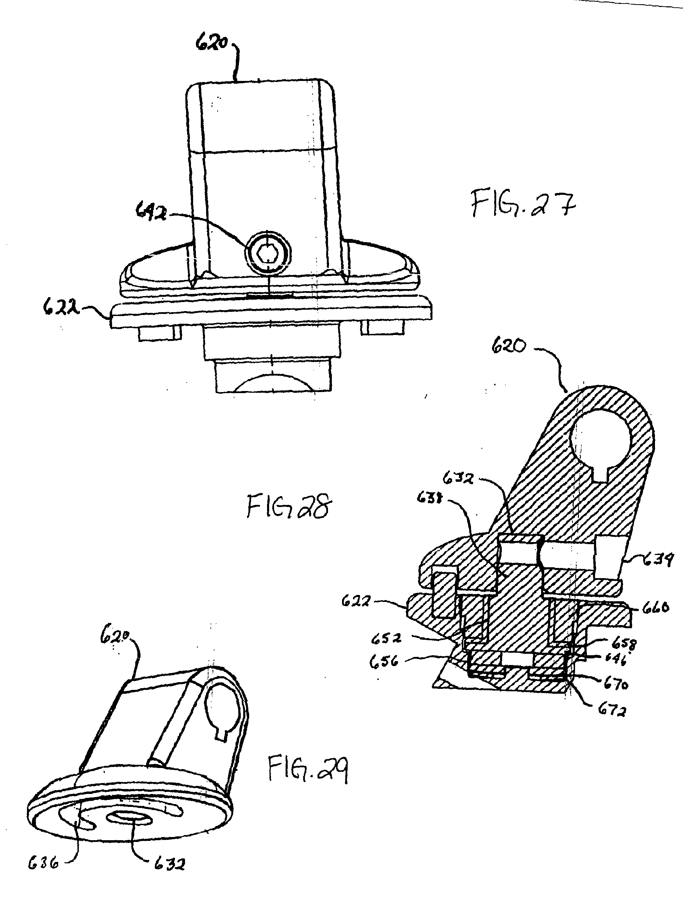

FIG. 27 is a side view of an alternative embodiment of the boom joint pivot 620 and an alternative embodiment of the mast top plug 622 shown in FIG. 21. In FIG. 27, the side of the boom joint pivot 620 having the screw 642 is shown. The boom joint pivot 620 may be mounted on the mast top plug 622 as described above. The screw 642 may attach the boom joint pivot 620 to the pivot shaft 638 (as seen in FIG. 21) .

FIG. 28 is a side cross-sectional view of an alternative embodiment boom joint pivot 620 and an alternative embodiment mast top plug 622. The boom joint pivot 620 includes the vertical bore opening 632 and the horizontal bore opening 634. The boom joint pivot 620 further includes the arcuate slot 636 that receives the pin 666 set in the mast top plug 622. The pivot shaft 638 is in position in the vertical bore opening 632. The through-hole 640 in the pivot shaft 638 may be aligned with the horizontal bore opening 634, i.e., in position to receive the screw 642 (not shown).

The mast top plug 622 may be seen in greater detail than in FIG. 21. The generally cylindrical opening 652 axially is shown to be located and extending from the upper surface 654 of the mast top plug 622. The opening 652 may receive elements of the inventive damp that may enable the boom joint pivot 620 to pivot with respect to the mast top plug 622 but may also damp the rotation of the boom joint pivot 620. As can be seen in FIG. 21, the opening 652 may be configured to receive the friction pad 656, the bushing 658, and spanner nut 660. The friction pad 656 may provide greater frictional resistance with increasing compressive force against it. The friction pad 656 may be, for example, made of an elastomeric material, such as those materials suitable for the friction washer, as described above. The bushing 658 may be, for example, made of a material having a low coefficient of friction such as a plastic material including those described above for use in the bushing 254 in FIG. 9. A washer 670 and a spring 672 such as a Belleville washer may be located between the friction pad 656 and the bottom of the opening 652.

As described above, the spanner nut 660 may be screwed down against the bushing 658 which may press down on the flange 646 of the pivot shaft 638. The flange 646 of the pivot shaft 638 contacts the friction pad 656. The spring 672 provides force pressing the friction pad against the flange 646 from below. The pivot shaft 638 is rotatable within the bushing 658 while the friction pad 656 damps the rotation of the pivot shaft 638. The spanner nut 660 enables the damping of the rotation of the pivot shaft 638 to be adjustable as the damping may be increased when the spanner nut 660 is screwed down more tightly on the pivot shaft 638.

FIG. 29 is a perspective view of the alternative boom joint pivot 620. The arcuate slot 636 that receives the pin 666 may be clearly seen as is the vertical bore opening 632.

FIG. 30 is a perspective view of the vertical pivot shaft 638. The vertical pivot shaft includes the first portion 648 having a smaller diameter than the second portion 650. The flange 646 is contiguous to the second portion 650. The through-hole 640 may be a horizontal bore through the first portion 648 of the vertical pivot shaft 638. The vertical pivot shaft 638 also includes a flange 646 contiguous to the second portion 650 of the vertical pivot shaft 638. The vertical pivot shaft 638 is, for example, made of metal or ceramic or polymeric material, including, among others, those suitable for the boom and mast as described above.

FIG. 31 is a cross-sectional view of the pivot shaft 638. The pivot shaft 638 includes the first portion 648 having a smaller diameter than the second portion 650. The flange 646 is contiguous to the second portion 650. The through-hole 640 may be shown having one chamfered end 680. Alternatively, both ends of the through-hole 640 may be chamfered.

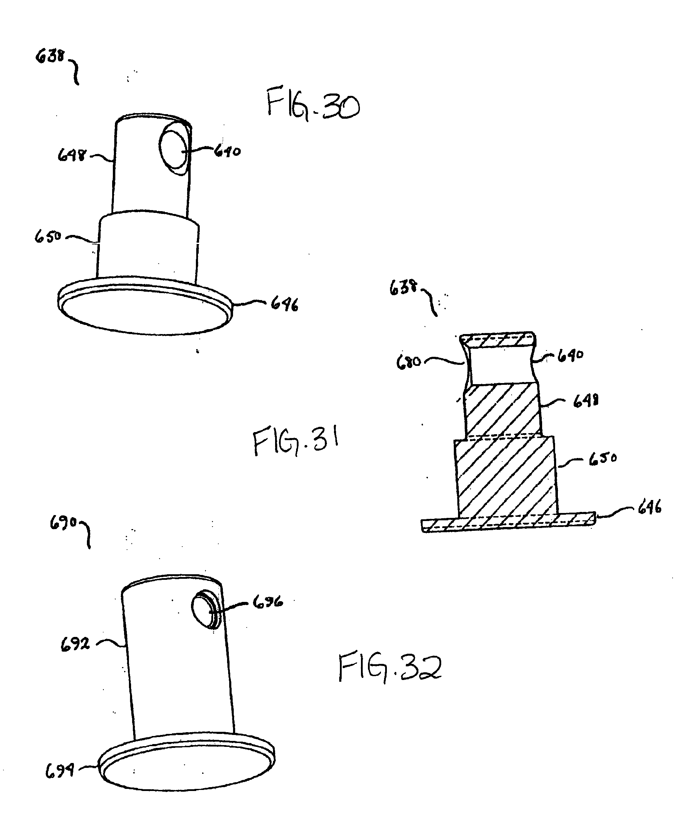

FIG. 32 is a perspective view of a pivot shaft according to another embodiment of the invention. The pivot shaft 690 includes a cylindrical portion 692 contiguous with a flange 694. The cylindrical portion 692 includes a through-hole 696. The through-hole 696 is shaped and configured to receive the screw 642 (not shown).

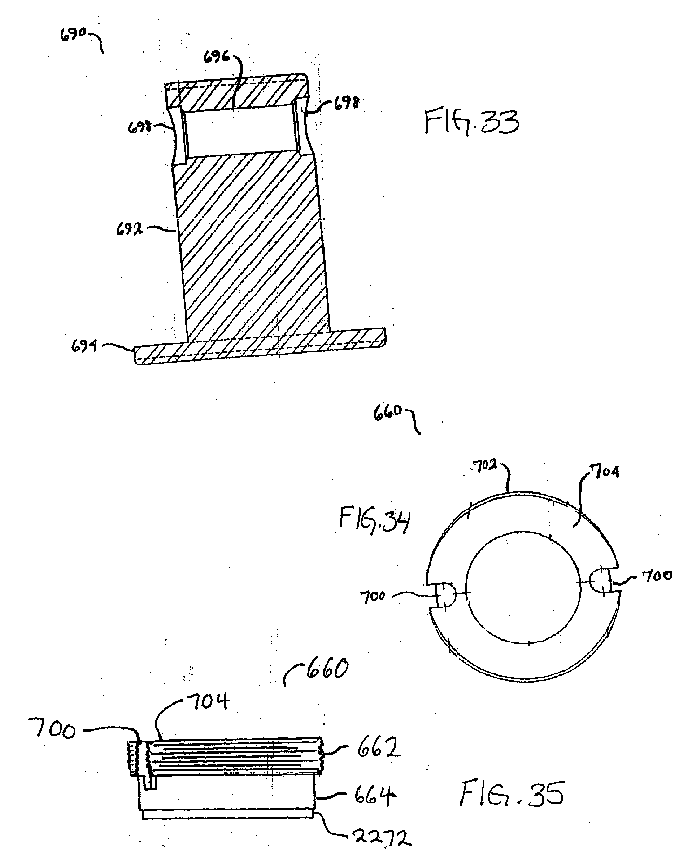

FIG. 33 shows, in cross-section, the pivot shaft 690, as shown in FIG. 32. As described above, the pivot shaft 690 includes the cylindrical portion 692, the flange 694 and through-hole 696. In this view, the through-hole 696 is counter-sunk and chamfered at both ends 698.

FIG. 34 shows, in top view, the spanner nut 660. The spanner nut 660 may also be referred to as a compression plug. The spanner nut 660 may be substantially circular in shape. In one embodiment, the spanner nut 660 may have two curved notches 700 located symmetrically about the circumference 702 of the spanner nut 660. The notches 700 extend from the upper surface 704 of the spanner nut 660 and end at a point before the bottom surface (not seen in this view). The notches 700 may be shaped and configured to receive pins to prevent the spanner nut 660 from unscrewing from the opening of the mast top plug 622. In an alternate embodiment, the spanner nut 660 may only have one curved notch. In a further alternate embodiment, the spanner nut 660 may have several curved notches, typically symmetrically placed around the circumference of the spanner nut 660.

FIG. 35 shows, in side view, the spanner nut 660 according to principles of the invention. The spanner nut 660 includes the upper threaded portion 662 and the lower unthreaded portion 664. One notch 700 can be seen extending from the upper surface 704 of the spanner nut 660 and ending in the lower unthreaded portion 664.

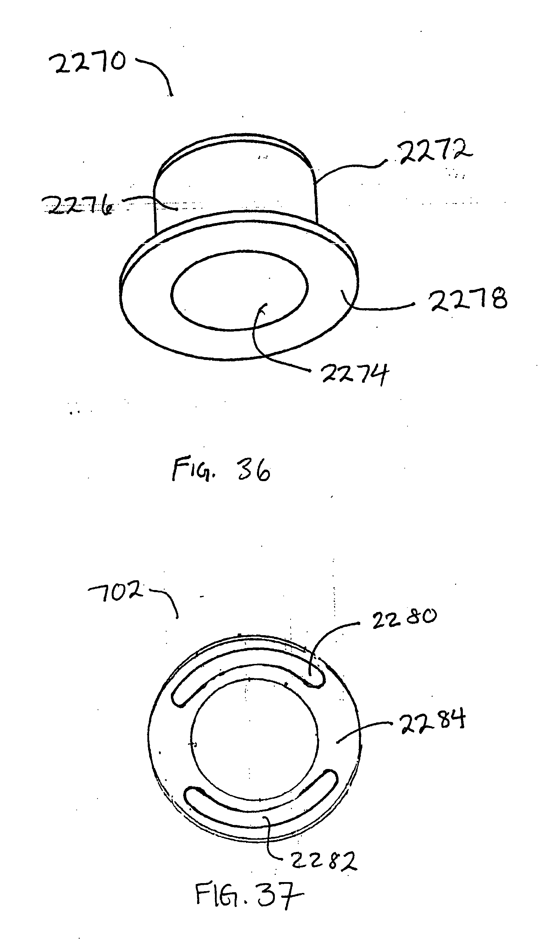

FIG. 36 shows a bushing 2270. The bushing 2270 may include a tubular member 2272 with inner 2274 and outer 2276 substantially cylindrical surfaces disposed in coaxial spaced relation to one another. The bushing 2270 also may include a flange portion 2278 disposed radially outwardly from outer surface 2276.

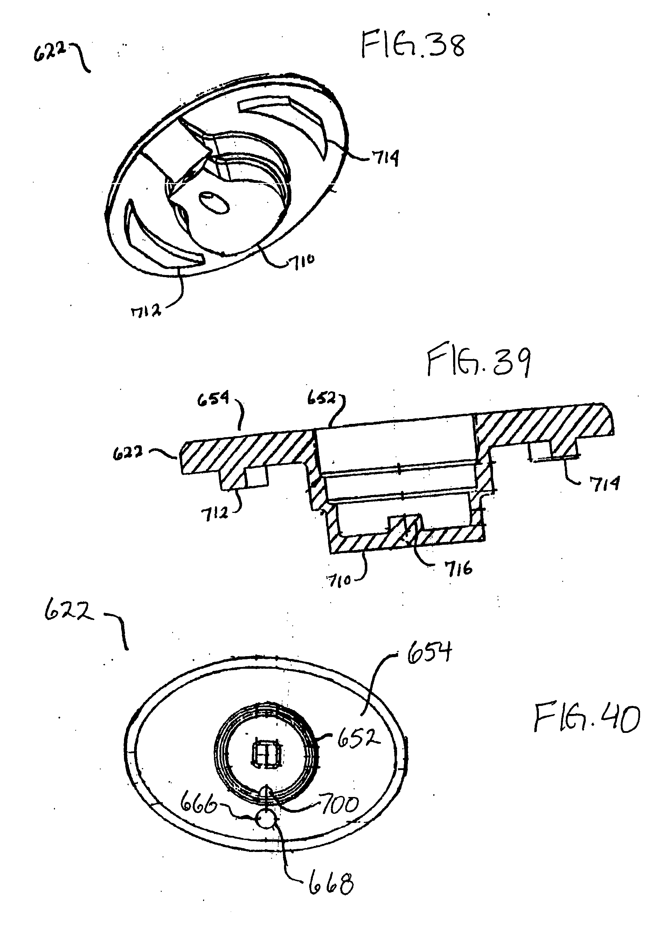

FIG. 37 shows a spanner nut 660 according to a further embodiment of the invention. The spanner nut 660 includes first 2280 and second 2282 arcuate slots in a upper surface 2284. The slots 2280, 2282 are adapted to receive a tool for rotation of the spanner nut 660. In contrast to the slots 700 of the spanner nut 660 shown in FIG. 34, slots 2280 and 2282 do not interrupt the threads 700 of the nut. Accordingly, the nut of the FIG. 37 embodiment may be less likely to experience cross-threading or other damage during installation than the spanner nut 660 of FIG. 34.

FIG. 38 is a perspective view of the mast top plug 622 according to principles of the invention. The mast top plug 622 includes a generally cylindrical-shaped structure 710 substantially centered in the mast top plug 622. A first arc-shaped structure 712 and a second arc-shaped structure 714 are disposed on either side of the center structure 710. The center structure is shaped and configured to hold the pivot and friction pad assembly described above. The first and second arc shaped structures 712, 714 serve to align the mast top plug 622 within the hollow bore in the mast 101.

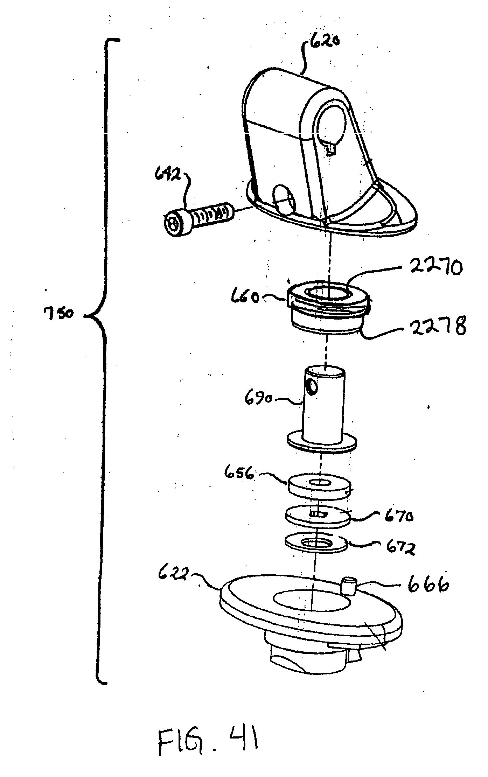

FIG. 39 is a cross-sectional view of the mast top plug 622. The center structure 710 of the mast top plug 622 includes the generally cylindrical opening 652 axially located and extending from the upper surface 654 of the mast top plug 622. The opening 652 is configured to receive the spring 672, washer 670, friction pad 656, pivot shaft 638, bushing 658 and spanner nut 660 as described above. A protrusion 716 in the bottom of the opening 652 enables alignment of the spring and washer. The opening 652 is threaded near the upper surface 654 to mate with the threads of the spanner nut 660. The mast top plug 622 further includes the first arc-shaped structure and the second arc-shaped structure that are used to align the mast top plug 622 in the mast 101.

FIG. 40 shows a top view of the mast top plug 622 according to principles of the invention. The top of the mast top plug 622 is oval-shaped in the present embodiment of the invention to match the oval configuration of the mast 101. The upper surface 654 of the mast top plug 622 is substantially flat. The mast top plug 622 includes an opening 652 extending from the upper surface 654 of the mast top plug 622. The spanner nut 660 is in position in the opening 652. The spanner nut 660 includes a notch 700. The notch 700 receives a pin (not shown) that prevents the spanner nut 660 from moving once the spanner nut 660 is screwed down into the opening 652 to the desired position. The mast top plug 622 further includes a bore opening 668 including a pin 666.

FIG. 41 shows, in assembly view, a mast-top plug and pivot assembly 750 according to another embodiment of the invention. The assembly includes the pivot 620 and the spanner nut 660, the pivot shaft 690, the friction pad 656, the washer 670, the spring 672, the mast top plug 622 and the screw 642. The bushing 658 is located inside the spanner nut 660.

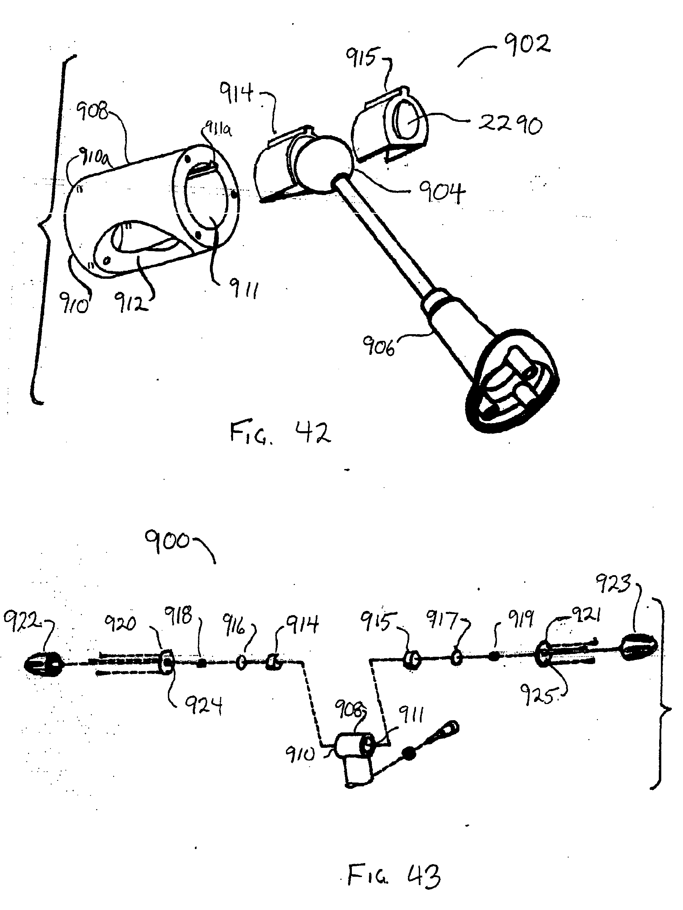

FIG. 42 shows, in perspective view, components of a ball and socket joint according to one embodiment of the invention. The ball and socket joint (also referred to as a ball joint) 902 includes a head tube 908 having a first opening 910 and a second opening 911 at opposite ends of the head tube 908. The openings 910, 911 also include grooves 910a, 911a respectively. A third opening 912 is present in the side of the head tube 908. The ball joint 902 further includes a first ball cup 914 and a second ball cup 915 to be received into the first and second openings 910, 911, respectively. A pivot mount 906 that holds the dental whitening lamp head (not shown) connects to the ball joint 902 by a ball swivel 904. The first and second ball cups 914, 915 are configured to receive the ball swivel 904 through the third opening 912 in the head tube 908. The ball joint 902 will be described in more detail below.

According to one embodiment, each ball cup 915 may include a contact plate 2290. In various embodiments of the invention, the reinforcing plate may be integrally molded with the ball cup 915, or coupled to a surface of the ball cup 915 with an adhesive or an adhesive tape, or by a fastener such as a screw or rivet.

The first and second openings 910, 911 may be adapted to receive the first and second ball cups 914, 915 respectively. A pivot mount 906 that is adapted to hold the dental instrument or equipment connects to the ball joint 902 by a ball swivel 904. The first and second ball cups 914, 915 may be configured to receive the ball swivel 904 through the third opening 912 in the head tube 908. The ball joint 902 will be described in more detail below.

FIG. 44 shows, in sectional perspective view, components of a ball joint (also referred to as a ball joint) according to one embodiment of the invention. The ball joint 902 is shown with a cutaway view of the head tube 908. The first ball cup 914 is in place inside the head tube 908. The ball swivel 904 of the pivot mount 906 is shown inserted through the third opening 912 of the head tube 908.

One of skill in the art will appreciate that a ball joint, such as that illustrated, for example, in above FIGs. 42 and 44, is merely exemplary of the various formations or coupling features which may be used to couple a dental instrument or apparatus or device to an end of the boom 103. For example in an alternative embodiment a flexible member, such as a gooseneck member, is disposed between the payload apparatus and the anterior end of the boom. The support structure of invention may include any flexible coupling device appropriate to a particular application and payload apparatus.

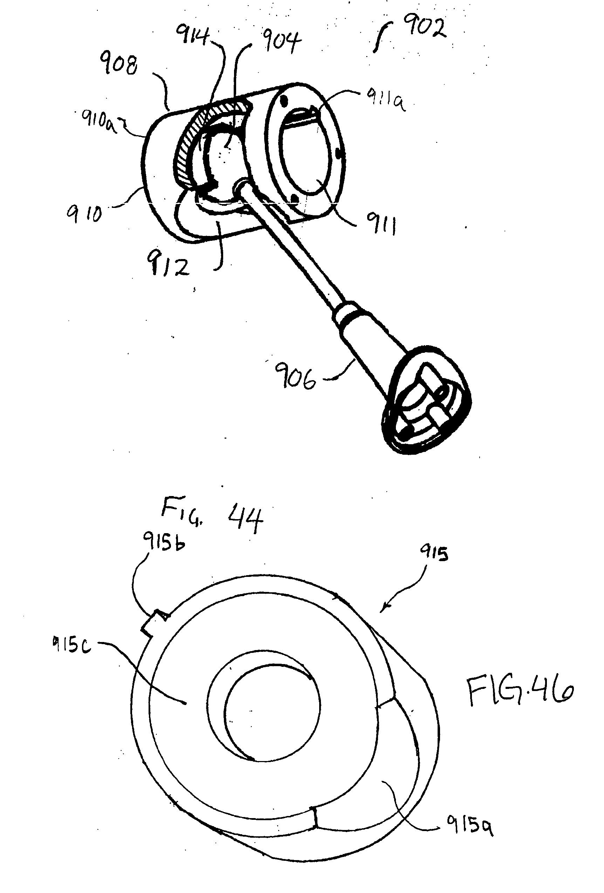

FIG. 43 shows, in exploded perspective view, a forward assembly 900 for a dentistry equipment support structure boom, enabling separable attachment between the instrument (not shown here) and the boom 103. The head tube 908 may be attached to the end of the boom 103. The instrument connector assembly 900 may be a socket joint including a ball joint 902 adapted to receive the ball swivel 904 of pivot mount 906 on the supported equipment as shown, for example, in FIG. 44.

The head tube 908 may be attached to the end of the boom 103. The head tube 908 has three openings, the first and the second opening 910, 911 on each end of the tube to receive the pieces that create the ball joint and the third opening 912 at the front of the head tube 908 to receive the ball swivel 904 on the dental equipment to be supported (not shown).

The forward assembly 900 of the present embodiment includes a first and a second ball cup 914, 915, a first and a second spacer 916, 917, a first and a second spring 918, 919, a first and a second nut plate 920, 921 and a first and a second ball joint knob 922, 923. Each ball cup 914, 915 has a curved surface so that when the ball cups 914, 915 are mated at the curved surfaces a substantially spherically-shaped space configured to receive the ball swivel 904 is formed.

To form the ball joint, the ball cups 914, 915 are inserted into the head tube 908 so that the spherically-shaped space aligns with the third opening 912 of the head tube 908. The spacers 916, 917 are inserted into openings 910 and 911 respectively and positioned on either side of the mated ball cups 914, 915. The first and second springs 918, 919 are placed against the first and second spacers 916, 917 respectively.

The nut plates 920, 921 are attached on opposing ends of the head tube 908 over the first and second openings 910, 911. The nut plates 920, 921 each may have a central opening 918, 919 that may be threaded.

According to one embodiment of the invention, each of the knobs 922, 923 may include an ultrasonically welded stud having an externally threaded distal end. The screws of the knobs 922, 923 are screwed through the central openings of the nut plates 920, 921 and press against the springs 918, 919, spacers 916, 917, and ball cups 914, 915 to press the ball cups 914, 915 against the ball swivel 904. When the knobs 922, 923 are tightened down, the received ball swivel 904 may not move inside the mated ball cups 914, 915. When the knobs 922, 923 are loosened, the received ball swivel 904 may move inside the mated ball cups 914, 915.

A first alternative embodiment of the ball socket involves relying on spring strength rather than pressure from a screw to put pressure against the ball cups 914, 915. Further, the springs 918, 919 shown here are coil springs. Alternatives to coil springs include, for example, spring washers, and other mechanisms for applying linear force, as known to those of skill in the art.

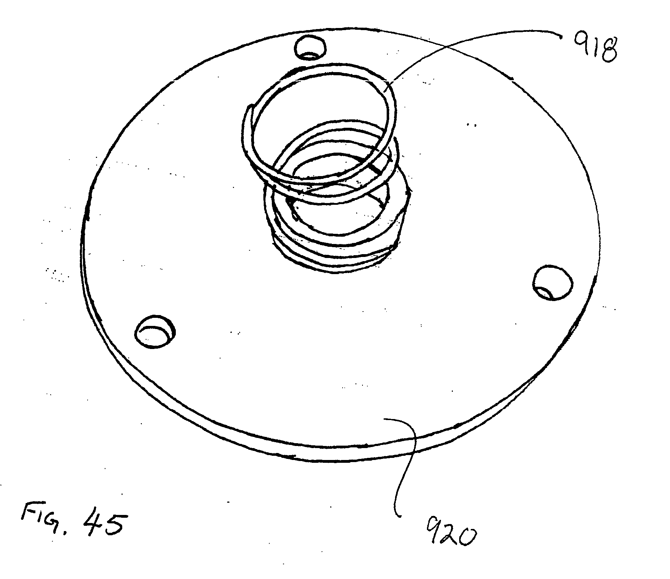

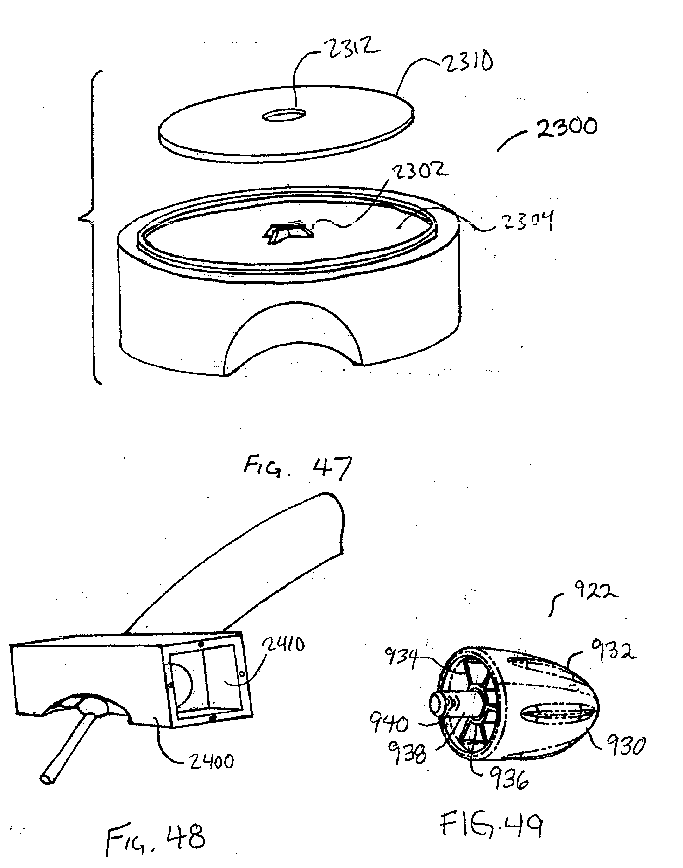

FIG. 45 shows, in additional detail, a nut-plate 920, 921 and spring subassembly 918 according to one embodiment of the invention. The spring assembly is adapted for supplying the spring tension for compressing the ball cups 914, 915 in response to the rotations of the absent knobs 922, 923, allowing the freedom of movement of the ball swivel 904 to be varied.. FIG. 46 shows an enlarged view of the ball cup 915, which is a mirror image of the ball cup 914. The ball cup 915 includes an internal space 915c that, when mated with the corresponding space in ball cup 914, forms a substantially spherical space that may retain the ball swivel 904 (as shown in FIG. 44). An aperture 915a allows the shaft connecting to the ball swivel 904 to exit the head tube 908 through opening 912 (as shown in FIG. 44). The ball cup 915 also includes a ridge 915b that restricts the orientation of insertion as well as rotation of the ball cup 915 in the opening 911 of head tube 908 by fitting into groove 911a (as shown in FIG. 42).

FIG. 47 shows another embodiment of a ball cup 2300 according to another embodiment of the invention. During assembly of the assembly 900 (as shown in FIG. 43), the contact plates 2290 must be mounted to the ends of the ball cups 914, 915. This may be achieved by using a double sided adhesive tape or other forms of adhesives. In the embodiment depicted in FIG. 47, the contact plate 2310 includes a central hole 2312. During mounting of the contact plate 2310 to the ball cup 2300, the central hole may come to rest upon the formation 2302 on the mounting surface 2304 of the ball cup 2300. The formation 2302 may be of any shape, such as a cross or other multi-pointed form, but is slightly larger in width than the diameter of the central hole 2312 of the contact plate 2310. To achieve fixture of the contact plate 2310, compression may be used to force the formation 2302 to fit within the confines of the central hole 2312. This fit may be a compression or frictional fit, such that the contact plate 2310 is substantially fixed to the ball cup 2300.

FIG. 47 shows an alternative embodiment of a ball joint according to another embodiment of the invention. The head tube 2400 may serve substantially the same purpose as the head tube 908 of FIG. 42, but is of square, rectangular or other angled shape in cross-section, rather than circular or elliptical as in FIG. 42. The angled shape of the opening 2410 allows a ball cup to be inserted in a similar manner as that depicted in FIG. 42, however a ridge, such as the ridge 915b shown in FIG. 46 and its corresponding groove 911a are not necessary to prevent rotation of the ball cup within the head tube due to the irrotatable nature of angled fits.

One of skill in the art will appreciate that a ball joint, such as that illustrated, for example, in FIGs. 42-44, is merely exemplary of the various coupling features which may be used to couple a dental apparatus or device to an end of the boom 103. For example in an alternative embodiment a flexible member, such as a gooseneck member, is disposed between the payload apparatus and the anterior end of the boom. The support structure of invention may include any flexible coupling device appropriate to a particular application and payload apparatus.

FIG. 49 shows a ball joint knob 922, according to one embodiment, in additional detail. The ball joint knob includes a body having a semi-ovoid external surface 930. The interior of the ball joint knob 922 is partially hollow, and includes a plurality of ribs 934 disposed between an inner cylindrical member 936 and an inner surface of the body. The inner cylindrical member has a first longitudinal axis, and a shaft 938 with a second longitudinal axis is disposed within the inner cylindrical member 936, such that the first and second longitudinal axes are substantially coincident. As illustrated, the shaft includes a substantially cylindrical outer surface bearing a plurality of threads 940 on a portion thereof. The threads 940 are adapted to be coupled to internal threads 924 of a corresponding nut-plate 920, as shown in FIG. 45.

The various inventive embodiments of a dentistry support structures heretofore described will be understood by one skill in the art to be adaptable to the support of a wide variety of conventional and novel dentistry apparatus. The resulting novel combinations provide not only effective dentistry related functionality, but our efficient in that they allow for the interchange of functional modules and reuse of the support structure for a wide variety of procedures and functions.

Thus, one of skill will appreciate that a dentistry support structure according to the present invention is used in various embodiments to support a dental whitening lamp, a dental composition during lamp, a dental imaging system for endoscopic imaging as in, for example, endoscopic root planing, an ultrasonic imaging system, and a support system for x-ray film and/or electronic x-ray sensors adapted for use in x-ray imaging of dental x-ray-graphic subjects.

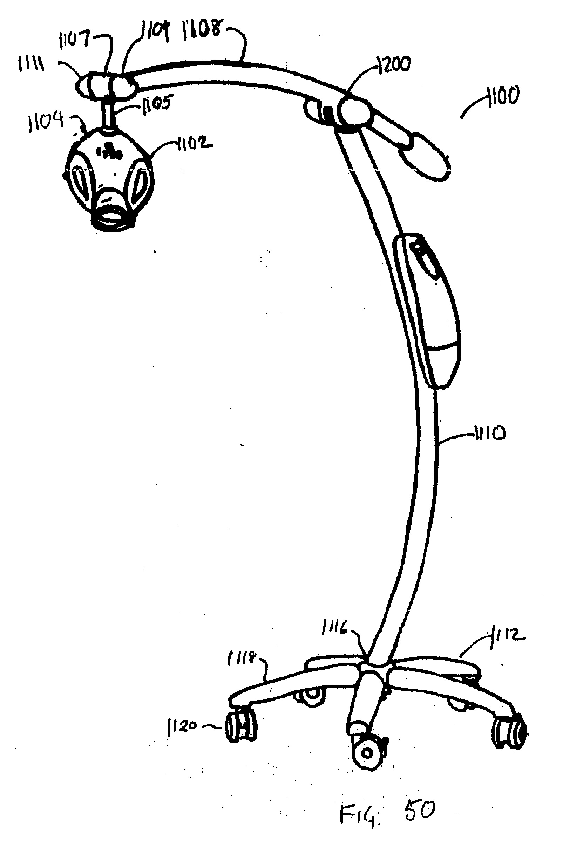

FIG. 50 shows a support structure for dental equipment according to one embodiment of the invention. The illustrated embodiment includes a dental whitening lamp 1100. The dental whitening lamp has a lamp head 1102 with a housing 1104. The lamp head 1102 also has a shaft 1105 that serves to couple the lamp head to a ball joint 1107. The ball joint 1107 is, in turn, coupled to a distal end of a boom 1108. A mast 1110 supports the boom 1108 and lamp head 1102. The mast 1110 is coupled at its upper end to the boom 1108 by a boom joint 1200. The mast 1110 is, in turn, supported at its lower end by a base 1112. In the illustrated embodiment, the base 1112 includes a plurality of arms 1118 extending from a center 1116 where the mast 1110 is attached. A caster wheel 1120 is attached to the end of each of the plurality of arms 1118 away from the center 1116. The caster wheels 1120 contact the floor thereby support the entire dental whitening lamp.

In the illustrated embodiment, the lamp head 1102 is adapted to be removably coupled to the body of a patient. In one exemplary embodiment, a lip retracting device, such as a lip retractor, is connected to a light guide that is in turn coupled to the lamp head 1102. Consequently, the present invention allows the lamp head to be supported in a way that allows its position to be extensively adjusted, so that the lamp head may be properly aligned with the patient for the dentistry process. This alignment will be maintained, even allowing for slight movement of the patient's head. This is possible because the lip retracting device is removably engaged with the light guide, and is positioned but not fixed to allow for this slight movement of the patient's head.

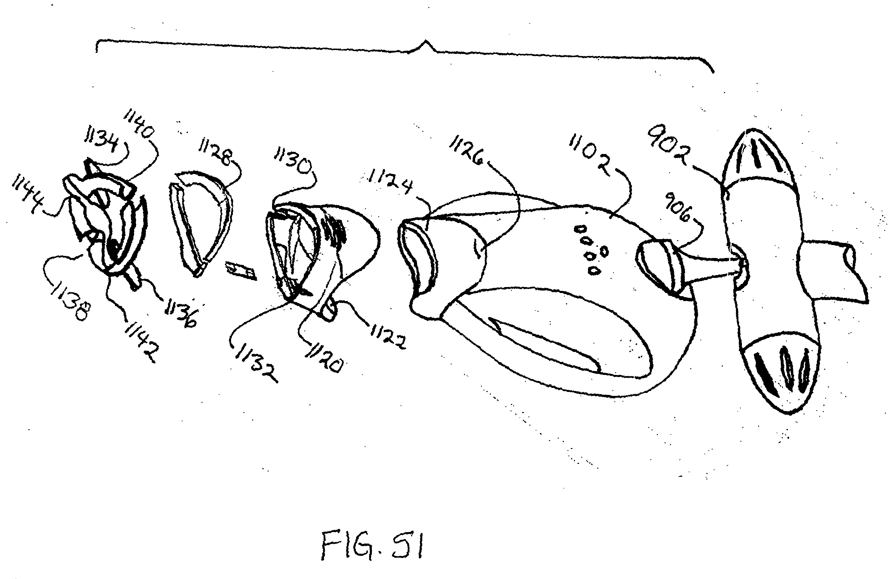

FIG. 51 shows an assembly relationship between the ball joint 902 the lamp head 1102, a light guide 1120, and a lip retractor device 1122 according to one embodiment of the invention. A pivot mount 906 is coupled between the lamp head 1102 and the ball joint 902. The ball joint allows the lamp head to be swiveled in space such that an optical axis of the curing lamp is aligned with the target teeth of a dental whitening subject.

A light guide 1120 is adapted to be coupled to an anterior end of the lamp head 1102. In one embodiment, the light guide 1120 includes an inner surface region 1122 that is adapted to be held in proximity to an outer surface region 1124 of the lamp head 1102. According to one embodiment of the invention, a projecting member, or bump, on inner surface 1122 is adapted to be urged into a recessed region 1126 of outer surface region 1124 so as to maintain the proximity of surface regions 1122 and 1124.

In one embodiment of the invention, the light guide 1120 includes an elastically compressible cushion 1128 at an anterior edge thereof. The elastically compressible cushion 1128 serves to soften an interface between a dental whitening process subject (not shown) and the light guide.

In a further aspect of the invention, as shown in the illustrated embodiment, the light guide includes first and second slots 1130 and 1132. These slots are adapted to receive projecting wings 1134, 1136 of a lip retractor 1138 so as to stabilize a relationship between the dental whitening subject and the lamp head.

The lip retractor 1138 includes channels 1140, 1142 adapted to support the lips of a dental whitening subject during the whitening process, and an elastic member 1144. The elastic member 1144 is coupled to the channels 1140, 1142 and adapted to urge the channels outwardly towards the lips, so as to couple the whitening subject to the lip retractor.

When the whitening subject is coupled to the lip retractor 1138, and the lip retractor is coupled to the light guide 1120 by the insertion of wings 1134, 1136 in respective slots 1130, 1132, the whitening subject is spatially stabilized with respect to the lamp head 1102. In this way the support structure serves to support the lamp head in a substantially stable spatial relationship to the whitening subject.

As discussed above, this spatially stabilized relationship between a subject and the support structure of the invention is found in other embodiments of the invention and in relation to various apparatus and processes.
In one embodiment, input/output cables may be used to provide a first electrical connection between the light source to the power pack and power cables to provide a second electrical connection between the power pack and an external power source, such that the input/output cables and the power cables are removably attached to the power pack.

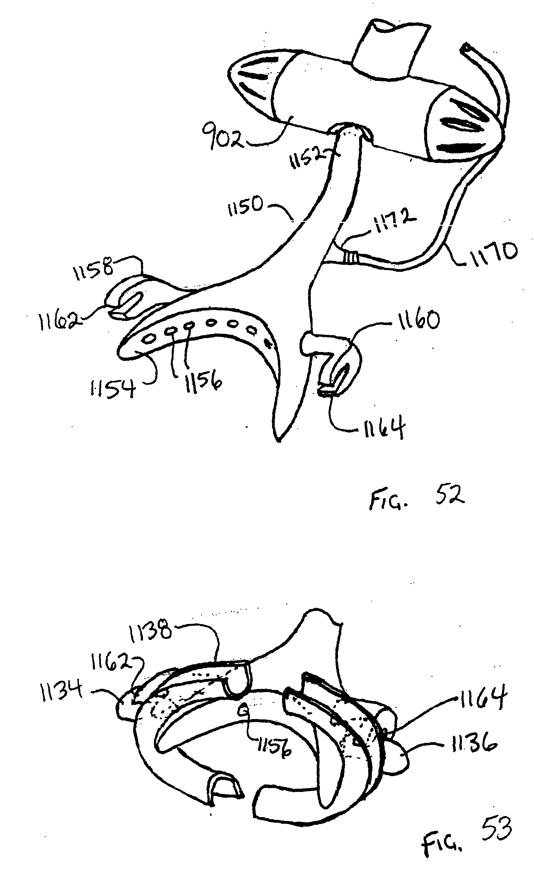

FIG. 52 shows a dental illumination source according to another embodiment of the invention. In the illustrated embodiment, the dental illumination source is a dental whitening illumination source. The reader will appreciate, however, that a dental composition curing source might equally well be shown. In the illustrated embodiment, the ball joint 902 is coupled to a light housing 1150. The light housing 1150 includes a first elongate portion 1152 having at its posterior end the ball of the ball and socket joint 902. An anterior end of the housing 1150 includes, in the illustrated embodiment, an arcuate surface 1154.

Arcuate surface 1154 support a one or more illumination sources 1156. In one embodiment of the invention the one or more illumination sources 1156 includes one or more light emitting diodes (LEDs). In another embodiment of the invention, the one or more illumination sources 1156 includes one or more miniature arc lamps such as, for example, halogen arc lamps. In still other embodiments of the invention, the one or more light sources 1156 includes one or more incandescent lamps such as, for example, halogen incandescent lamps, and in still other embodiments of the invention, the one or more light sources includes one or more optical fibers coupled to a remote light source and/or one or more optical wavelength transformer such as those described in United States patent application number 60/658,517, the disclosure of which is herewith incorporated by reference in its entirety.

In the illustrated embodiment, a signal cable 1170 is coupled at one end to the light housing 1150. The signal cable may include a power cable adapted to provide power for the one or more illumination sources 1156. The single cable may also include an optical light guide such as an optical fiber adapted to transmit light to the one or more illumination sources from a remote light source. In at least one embodiment of the invention, the signal cable 1170 includes a strain-relief feature 1172.

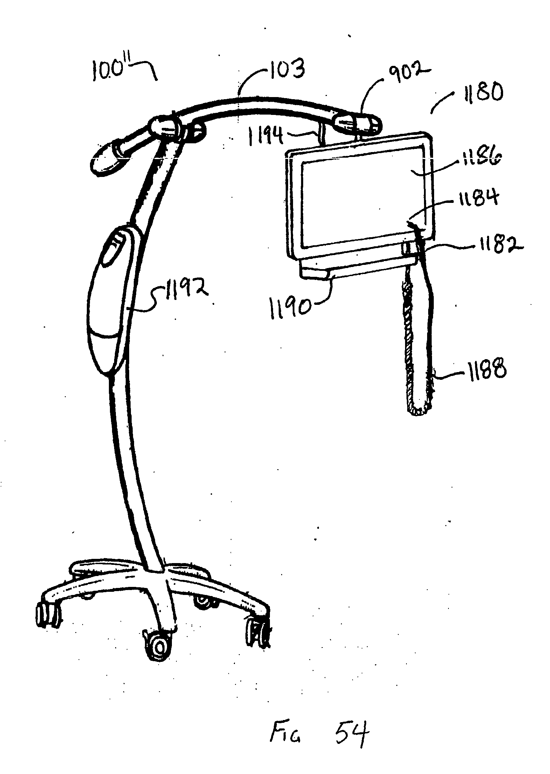

In one embodiment, the power cables may provide a connection from a power source external to the dental instrument and the power pack, and the power cables are substantially contained in the mast, and are removable from the external power source.
Illustrating another aspect of the invention, the embodiment of FIG. 52 shows first and second wing-coupling members 1158, 1160. Each wing-coupling member 1158, 1160 includes a respective slot 1162, 1164. The slots 1162, 1164 are adapted to receive corresponding wings 1134, 1136 of a lip retractor 1138, as illustrated in FIG. 53.

When the lip retractor 1138 is worn by a dental procedure subject, insertion of the wings 1134, 1136 into the slots 1162, 1164 serves to stabilize a spatial relationship between the subject and the one or more illumination sources 1156.

FIG. 54 illustrates a support structure for dentistry according to another embodiment of the invention. In FIG. 54, the support structure 100" is shown coupled to components of an endoscopic endodontic apparatus 1180. The apparatus 1180 of the illustrated embodiment is an endoscopic root planer. One of skill in the art will appreciate that it is known to perform an endodontic procedure in which the soft gum tissue of a patient is detached and drawn away from the patient's teeth to expose the roots of the teeth. Thereafter, a dental instrument such as a scaler is used to remove deposits of plaque or other material from the exposed roots. Ultimately, the patient's gum tissue is repositioned over the roots, sutured in place, and allowed to heal. Unfortunately, because of the invasive nature of the procedure the healing process tends to be lengthy and painful.

In the embodiment of the present invention illustrated in FIG. 54, the support structure 100" supports an endoscopic root planing system 1180. The endoscopic root planing system 1180 includes a handpiece 1182. The handpiece has a distal end 1184 that includes a root planing tool and an optical device.

The optical device is adapted to illuminate a small spatial region adjacent a tip of the root planing tool and receive reflected light. The reflected light received by the optical device is, in turn, received by a sensor either directly, or by way of an optical waveguide such as an optical fiber.

The sensor converts the received light into a signal that is amplified and displayed as an image on a display screen 1186 of the system. Although the handpiece 1182 is shown coupled to the balance of the root planning system 1180 by an umbilical cable 1180, a wireless handpiece is also within the scope of the invention disclosed herewith.

In one aspect of the invention, the root planing system of the invention is adapted to allow removal of plaque and other detritus from between the gum and root of a patient without surgical removal, and subsequent reattachment, of the gum.

In one aspect of the invention, the root planing system includes a tray 1190 adapted to the convenient storage of various dental instruments and/or materials. According to one embodiment of the invention, the tray 1190 is readily removable to allow sterilization of the tray as in, for example, an autoclave.

In another aspect of the illustrated embodiment, the monitor screen 1186 of the system is pivotally and/or removably coupled to the boom 103 of the support structure at ball joint 902. In still another aspect of the illustrated embodiment, the endodontic root planing system includes a power pack 1192. According to one embodiment of the invention, the power pack supplies operative electrical power to the balance of the root planing system by way of an electrical cable 1194. Still further embodiments of the invention include digital processing apparatus such as, for example, a microprocessor within the powerpack 1192. The digital processing apparatus is adapted to control and process signals of the endodontic root planning system.

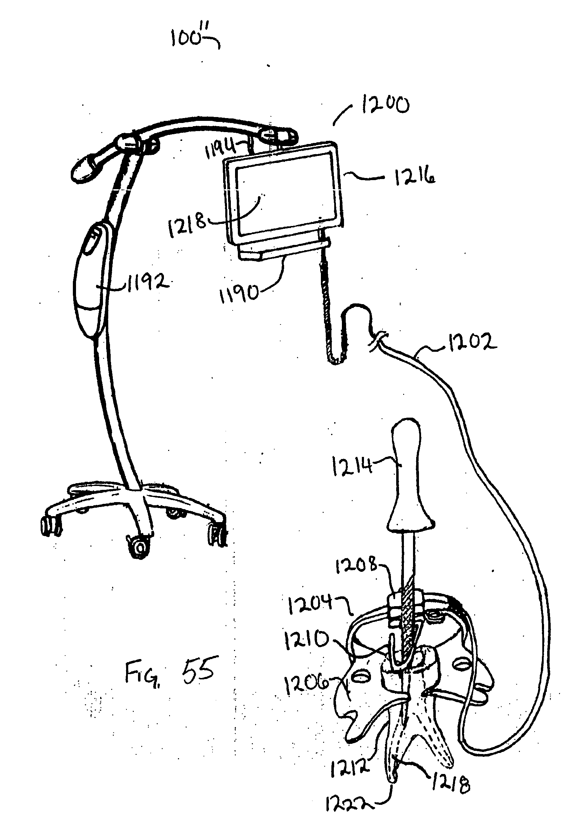

In the embodiment of the present invention illustrated in FIG. 55, the support structure 100" supports an endodontic apex locator system 1200. The endodontic apex locator system 1200 includes a signal transmission medium such as a coaxial signal wire 1202. The signal wire 1202 is adapted to be coupled to an apex locator fixturing device 1204.

The apex locator fixturing device 1204 includes a support clamp portion 1206, an insulator portion 1208 and a contactor portion 1210. The support clamp portion 1206 is adapted to be removably but firmly coupled to a tooth 1212 of a dental patient. The support clamp portion 1206 is adapted to support the insulator portion 1208 which, in turn, is adapted to support the contactor portion 1210.

During the performance of a root canal procedure, an endodontic file, reamer, or other appropriate tool 1214 is used to excavate a pulp chamber and root of the tooth 1212. A surface of the tool 1214 comes into contact with the contactor 1210 and an electrical signal received from the signal wire 1202 is electrically coupled to the tool 1214.

The signal wire 1214 is coupled at a second end to a processing device 1216 that is adapted to generate the electrical signal and produce a measurement based on, for example, an impedance of the tool 1214 and tooth 1212 system.

In response to the measured impedance, or other signal, the processing system 1216 produces an image on a display screen 1218 of the system. In various embodiments, the image is textural and/or graphical, and represents a spatial location of the tool 1214 with respect to a root canal 1218 of the tooth 1212. Although the apex locator fixturing device 1204 is shown coupled to the balance of the apex locator system 1200 by signal wire 1202, a wireless apex locator fixturing system is also within the scope of the invention disclosed herewith.

In one aspect of the invention, the apex locator system of the invention is adapted to allow excavation and shaping of the root canal 1218 without perforation of the root wall or of the portion of the periodontal membrane located at the apex 1222 of the root.

In one aspect of the invention, the apex locator system includes a tray 1190 adapted to the convenient storage of various dental instruments and/or materials. According to one embodiment of the invention, the tray 1190 is readily removable to allow sterilization of the tray as in, for example, an autoclave.

In another aspect of the illustrated embodiment, the monitor screen 1218 of the system is pivotally and/or removably coupled to the boom 103 of the support structure at ball joint 902. In still another aspect of the illustrated embodiment, the apex locator system 1200 includes a power pack 1192.

According to one embodiment of the invention, the power pack supplies operative electrical power to the balance of the apex locator system by way of an electrical cable 1194. Still further embodiments of the invention include digital processing apparatus such as, for example, a microprocessor within the power pack 1192. The digital processing apparatus is adapted to control and process signals of the endodontic apex locator system.

In one embodiment the invention includes the combination of a support structure 100" with an endodontic apex locator system such as that disclosed in United States patent application number 60/594,388 the disclosure of which is herewith incorporated by reference in its entirety.

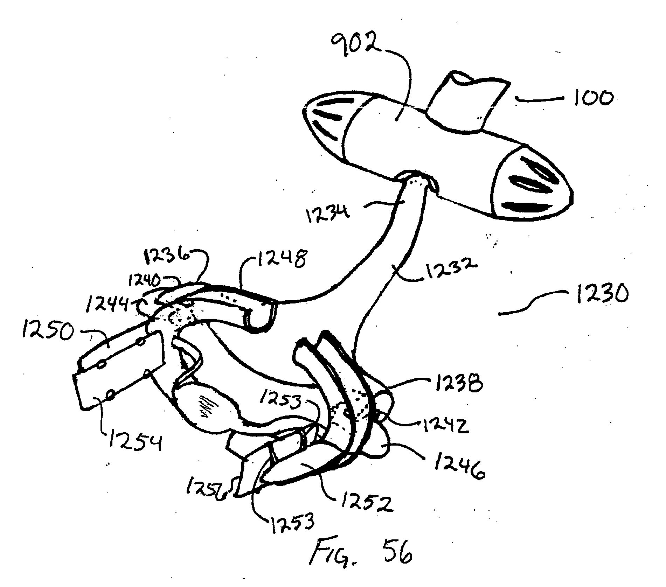

FIG. 56 shows another embodiment of the invention including the dental support structure 100 of FIG. 1 that is adapted to support a dental imaging fixturing system 1230. In the illustrated embodiment, the ball joint 902 is coupled to a support member 1232. The support member 1232 includes a first elongate portion 1234 having at its posterior end the ball of the ball and socket joint 902. An anterior end of the support member 1232 includes, in the illustrated embodiment, first and second wing-coupling members 1236, 1238. Each wing-coupling member 1236, 1238 includes a respective slot 1240, 1242. The slots 1240, 1242 are adapted to receive corresponding wings 1244, 1246 of a lip retractor 1248.

When the lip retractor 1248 is worn by a dental procedure subject, insertion of the wings 1244, 1246 into the slots 1240, 1242 serves to stabilize a spatial relationship between the subject and the one or more x-ray sources.

According to one embodiment of the invention, as illustrated, the lip retractor 1248 includes first and second bite members 1250, 1252. Each bite member 1250, 1252 has a respective one or more film support clips 1253 adapted to support a respective x-ray film package.

In operation, at least one x-ray film package is coupled to the one or more film support clips 1253. The x-ray film package, as is known in the art, includes a sheet of chemical x-ray film enclosed in a light-tight package. The lip retractor 1248 is coupled to a dental x-ray subject by placing the lips of the patient into the lip-receiving channels of the lip retractor 1248. The subject then bites down on the bite members to further secure the lip retractor in a stable spatial relationship to the teeth of the subject. By inserting the wings 1244, 1246 into slots 1240, 1242, the lip retractor 1248 is stabilized with respect to the dental support structure 100. This serves to stabilize the teeth of the subject and the x-ray film package 1256 with respect to the floor, and thus with respect to an x-ray source. Consequently, the well-known tendency of x-ray subjects to move during exposure of the x-ray film with a resulting nonuniformity of film exposure is reduced.

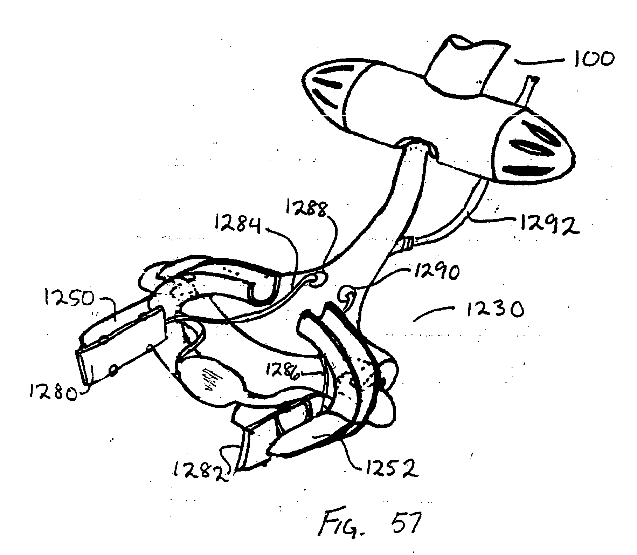

FIG. 57 shows a further embodiment of the invention including the dental support structure 100" of FIG. 54 that is adapted to support a dental imaging fixturing system 1230.

Unlike the embodiment of FIG. 56, the FIG. 57 embodiment includes electronic x-ray sensors 1280, 1282 coupled to the bite members 1250, 1252 respectively. Detecting and imaging x-rays with an electronic image sensor may be preferable to using chemical film because electronic image sensors tend to be more sensitive than chemical film, no chemical developing process is required, and the digital images produced by most electronic image sensors are immediately ready for digital manipulation.

In one embodiment of the invention, electronic image sensors each include a respective signal cable with a removable plug.

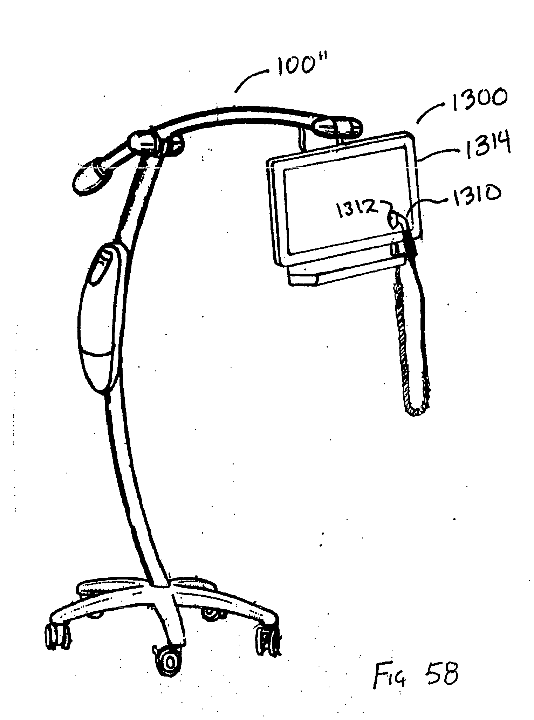

FIG. 58 shows the dental equipment support structure 100" of FIG. 54 including an ultrasonic imaging system 1300 according to one embodiment of the invention. The support structure includes an imaging handle 1310 that supports an ultrasonic transducer 1312, and an imaging screen 1314. The ultrasonic transducer is adapted to be positioned in proximity to a tooth and/or bone region. Ultrasonic vibrations generated and received by the transducer are used by the system 1300 to produce an image of the tooth and/or bone region on the screen 1314.

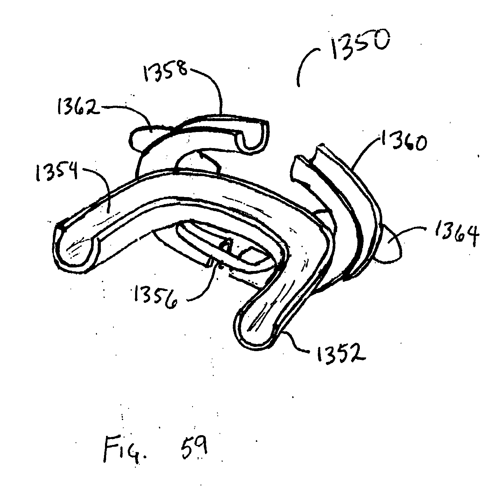

FIG. 59 shows a dental composition tray 1350 according to one embodiment of the invention. The dental composition tray includes first and second lip receiving channels 1358, 1360. A third tooth receiving channel 1354 is disposed in a substantially normal orientation to the lip receiving channels and adapted to receive, within a concave region thereof, the upper or lower teeth of a patient. First and second fixturing wings 1362, 1364 are coupled to the fist and second lip receiving channels 1358, 1360, respectively. As shown in the illustrated embodiment, a coupling member 1356 is disposed between, and mutually coupled to the first and second lip receiving channels 1358, 1360 and the tooth receiving channel 1352. One of skill in the art will appreciate that, in various embodiments, the dental composition tray includes a pair of tooth receiving channels arranged to receive both upper and lower teeth simultaneously.

In use, the dental composition tray 1350 is adapted to receive a dentistry composition, such as a dental whitening composition or a dental casting composition within the concave region 1354 of the tooth receiving channel 1352.

While exemplified embodiments of the invention have been described and illustrated above, it should be understood that these are exemplary of the invention and are not to be considered as limiting. Accordingly, the invention is not to be considered as limited by the foregoing description, but is only limited by the scope of the claims appended hereto. According to one embodiment of the invention, a friction washer used in the invention has an outer diameter of between about 0.50 inches and about 3 inches. In a particular embodiment, the friction washer has an outer diameter of about 0.78 inches. In another embodiment, a friction washer according to the invention has an outer diameter of about 1.91 inches. In still another embodiment, a friction washer according to the invention has a thickness of between about 0.01 inches and about 0.50 inches. In a particular embodiment, a friction washer according to the invention has a thickness of about 0.125 inches. One of skill in the art will appreciate that friction washers of other diameters, thicknesses and configurations will be applicable in various embodiments of the inventions, and fall within the scope of the invention as described herewithin. One of skill in the art will also appreciate that the foregoing measurements and ranges of measurements are intended to be understood as incorporating mechanical tolerances appropriate to the present invention.

## Claims

**1.** A dental support system comprising:
a pivot member comprising at least first and at least second formations, said at least first formation is adapted for inter-engaging at least one corresponding formation of at least a portion of dental instrument, to support said dental instrument when the pivot member and the at least a portion of the dental equipment are apposed; and
a base member comprising at least one formation adapted for inter-engaging said at least second formation of the pivot member;
wherein said pivot member is adapted for rotation about a substantially vertical axis with respect to said base member.

**2.** The dental support system of claim 1 wherein said pivot member comprises:
a boom comprising at least one formation about a section spaced away from its ends; and
a mast comprising at least one formation towards one end;
wherein said formation of said boom is adapted for inter-engaging said formation of said mast such that the boom is in a substantially perpendicular direction to the mast.

**3.** The dental support system of claim 1 or 2 wherein said formation on said base member comprises a pin, and said first formation comprises an arcuate slot adapted to receive said pin, said pin constrains movement of said pivot member about said axis.

**4.** The support system of claim 2 wherein said formation of said boom comprises a yoke and said formation of said mast comprises a shaft, said shaft being adapted to be substantially fixedly coupled to said mast.

**5.** The support system of claim 4 wherein said yoke comprising a first braking surface, said shaft comprising a second braking surface, said first and second braking surfaces being adapted to mutually engage a friction member.

**6.** The support system of claim 5 wherein said friction member comprises a friction washer, said friction washer including an internal surface defining a through-hole, said through-hole being adapted to receive a portion of said shaft therethrough.

**7.** The support system of claim 6 wherein said washer comprises an anti-rotation feature, said anti-rotation feature being adapted to minimize a rotational motion of said washer about a longitudinal axis of said shaft, whereby said washer is substantially rotationally fixed with respect to said mast.

**8.** The support structure of claim 6 or 7 wherein said anti-rotation feature comprises a first substantially flat region of said internal surface, and said shaft comprises an external surface including a second substantially flat region, said first substantially flat region being adapted to engage said second substantially flat surface.

**9.** The support system of claim 1, wherein said pivot member comprises:
a shaft, said shaft being mechanically coupled to said base; said shaft being substantially rotationally fixed with respect to said base, said shaft including a first plurality of threads on an external surface thereof, said shaft being adapted to support a yoke, said yoke being adapted to rotate about a longitudinal axis of said shaft, said yoke including a first braking surface;
a washer, said washer being adapted to be supported by said shaft such that said washer is substantially rotationally fixed with respect to said shaft, said washer including a second braking surface;
a friction member, said friction member being adapted to be disposed between said first and second braking surfaces; and
a knob, said knob including a second plurality of threads on an internal surface thereof, said first and second pluralities of threads being adapted to mutually engage one another;
whereby upon rotation of said knob about said longitudinal axis of said shaft in a given direction, said knob urges said first and second braking surfaces towards one another so as to mutually engage said first and second braking surfaces with said friction member.

**10.** A support system comprising:
a base comprising at least one formation;
a mast comprising at least first and at least second formations spaced apart from each other, said at least first formation is adapted for removably inter-engaging the formation of the base such that the mast is in a substantially perpendicular direction to the base; and
a boom having first and second ends and a central portion, said central portion comprising at least one formation adapted for inter-engaging said at least second formation of the mast for removably attaching to the mast in a substantially perpendicular direction to the mast.

**11.** The support system of claim 10 wherein said formation on said base member comprises a pin, and said first formation comprises an arcuate slot adapted to receive said pin, said pin constrains movement of said pivot member about said axis.

**12.** The support system of claim 10 or 11 wherein said mast comprises at least third formation spaced away from the first and second formations, said at least third formation is adapted to inter-engage at least one corresponding formation of a power pack.

**13.** The support system of claim 12 wherein said first end of the boom comprises at least one formation adapted for removably inter-engaging at least one corresponding formation of a dental instrument.

**14.** The support system of claim 12 or 13 wherein said first end of the boom comprises at least one formation adapted for removably inter-engaging at least one corresponding formation of a dental instrument.

**15.** The dental support system of claim 14 further comprising input/output cables to provide an electrical connection between the dental instrument and the power pack, said cables are substantially contained in the mast and the boom, and said cables in the boom are removably attached to the dental instrument and to the cables in the mast.

**16.** The support system of any of claims 10-15 wherein said formation of said boom comprises a yoke and said formation of said mast comprises a shaft, said shaft being adapted to be substantially fixedly coupled to said mast.

**17.** The support system of claim 16 wherein said yoke comprising a first braking surface, said shaft comprising a second braking surface, said first and second braking surfaces being adapted to mutually engage a friction member.

**18.** The support system of claim 17 wherein said friction member comprises a friction washer, said friction washer including an internal surface defining a through-hole, said through-hole being adapted to receive a portion of said shaft therethrough.

**19.** The support system of claim 18 wherein said washer comprises an anti-rotation feature, said anti-rotation feature being adapted to minimize a rotational motion of said washer about a longitudinal axis of said shaft, whereby said washer is substantially rotationally fixed with respect to said mast.

**20.** The support structure of claim 18 or 19 wherein said anti-rotation feature comprises a first substantially flat region of said internal surface, and said shaft comprises an external surface including a second substantially flat region, said first substantially flat region being adapted to engage said second substantially flat surface.

**21.** A dental support system for a dental illumination system comprises:
a rolling base for supporting the illumination system on a surface, the rolling base is adapted to position the illumination system proximate to a workpiece in a wide range of angles, and comprises a locking mechanism to substantially restricted the movement of the illumination system;
a mast attached to the rolling base;
a boom attached substantially perpendicular to the mast, said boom having rotational and tilt movement with respect to the mast; and
at least one illumination system attached to an end of the boom, said illumination system having rotational and tilt freedom of movement with respect to the end of the boom to facilitate tooth whitening procedure.

**22.** The dental support system of claim 21 wherein said system may be adapted to be positioned at the right or the left side of any workpiece.

**23.** The dental support system of claim 21 or 22 wherein the boom is curved.

**24.** The dental support system of claim 21, 22 or 23 further comprising at least one power pack attached to the mast.

**25.** A dental support system comprising:
a pivot member, said pivot member being adapted to support a dental instrument;
a base member, said base member being adapted to support said pivot member, whereby said pivot member may be rotated about a substantially vertical axis with respect to said base member.

**26.** The dental support system of claim 25 wherein said base member comprises a pin, and said pivot member comprises an arcuate slot adapted for receiving said pin such that said pin constrains movement of said pivot member about said axis.

**27.** The dental support system of claim 25 or 26 wherein the base member comprises a braking device, said braking device dampens the movement of said pivot member about said axis.

**28.** The dental support system of claim 25 or 27 wherein said pivot member comprises a boom and a mast.

**29.** The dental support system of claim 28 wherein said boom comprises a boom hinge positioning device for angularly positioning the mast with respect to the base member.

**30.** The dental support system of claim 29 wherein said boom hinge positioning device can be locked to hold the boom in position.

**31.** The dental support system of claim 29 or 30 wherein said boom hinge in a locked position is adapted to withstand a static load of about fifty pounds against a distal end of a boom connected to said boom hinge.

**32.** The dental support system of claim 30 or 31 wherein said boom hinge positioning device is adapted to withstand the force of gravity when a dental instrument is coupled to the boom at its distal end.

**33.** A dental support system comprises:
a pivot;
at least one substantially horizontal shaft supported by said pivot;
a yoke member pivotally supported by said shaft, such that said yoke member is adapted to pivot on a substantially horizontal axis;
a substantially vertical shaft coupled to said pivot; and
a plug member having a bore adapted to receive said substantially vertical shaft and an upper surface adapted to support said pivot.

**34.** The dental support system of claim 33 further comprising a friction member disposed between a surface of the pivot that moves with respect to a surface of the plug member.

**35.** The boom hinge of claim 33 or 34 wherein the friction member comprises a washer disposed between an upper substantially flat surface of the plug member and a lower substantially flat surface of the pivot, said flat surfaces are substantially parallel to each other and substantially perpendicular to the vertical shaft and are adapted to rotate with respect to each other about a vertical axis of the vertical shaft.

**36.** The dental support system of claim 33, 34 or 35 further comprising a friction device disposed between the pivot and the plug member, the friction device being adapted to damp rotational movement of the pivot with regard to the plug member.

**37.** The dental support system of claim 36 wherein the friction device is adapted for providing a variable amount of damping.

**38.** The dental support system of claim 36 or 37, wherein the friction device comprises:
a friction pad; and
a compression device to compress a rotational surface of the pivot against the friction pad.

**39.** The dental support of any of claims 33-38, further comprising a friction member disposed between the shaft and the plug member.

**40.** The dental support system of claim 39 wherein the friction member comprises of a rubber ring disposed on the shaft.

**41.** A dental support system comprising:
a first bearing adapted to pivot about a substantially horizontal axis of a yoke;
a second bearing adapted to pivot about a substantially vertical axis of a pivot member; and
a damper adapted to retard pivotal motion about said substantially vertical axis.

**42.** The dental support system of claim 41 wherein said yoke comprises a boom.

**43.** The dental support system of claim 41 or 42 wherein said pivot member comprises a mast.

**44.** The dental support system of claim 42 wherein said boom comprises at least one formation adapted to inter-engage a corresponding formation on the yoke.

**45.** The support system of any of claims 41-45 wherein said yoke comprising a first braking surface, said shaft comprising a second braking surface, said first and second braking surfaces being adapted to mutually engage a friction member.

**46.** The support system of claim 45 wherein said friction member comprises a friction washer, said friction washer including an internal surface defining a through-hole, said through-hole being adapted to receive a portion of said shaft therethrough.

**47.** The support system of claim 46 wherein said washer comprises an anti-rotation feature, said anti-rotation feature being adapted to minimize a rotational motion of said washer about a longitudinal axis of said shaft, whereby said washer is substantially rotationally fixed with respect to said mast.

**48.** The support structure of claim 46 or 47 wherein said anti-rotation feature comprises a first substantially flat region of said internal surface, and said shaft comprises an external surface including a second substantially flat region, said first substantially flat region being adapted to engage said second substantially flat surface.

**49.** A dental support system comprising:
a substantially horizontal shaft rotatably supporting a yoke member;
a substantially vertical shaft rotatably positioning a pivot member; and
a brake device adapted to damp a rotation of said pivot member about said substantially vertical shaft.

**50.** The dental support system of claim 498 wherein said brake device comprises a frictional member.

**51.** The dental support system of claim 49 or 50 wherein said frictional member comprises material selected from the group consisting of a synthetic elastomeric polymer, a natural elastomeric polymer, a viscous fluid and combinations thereof.

**52.** The dental support system of claim 49, 50 or 51 wherein said brake device comprises a compression plug adapted to urge a first friction surface of said frictional member against a second frictional surface.

**53.** The dental support system of claim 52 wherein said compression plug comprises a substantially cylindrical outer surface bearing a plurality of threads.

**54.** The dental support system of any of claims 49-53 wherein said brake device further comprises a spring to urge the first friction surface of said frictional member against the second frictional surface.

**55.** The dental support system of any claims 1-10, 13-20 and 25-31 wherein said dental instrument comprises a dental whitening system, a dental curing system, a dental examination system, a viewing and cleaning instrument; an imaging equipment; an X-ray equipment, a root canal apex locator, or combinations thereof.

**41.** A dental support system comprising:
a first bearing adapted to pivot about a substantially horizontal axis;
a second bearing adapted to pivot about a substantially vertical axis; and
a damper adapted to retard pivotal motion about said substantially vertical axis.

**42.** The dental support system of claim 41 wherein said substantially horizontal axis comprises a boom.

**43.** The dental support system of claim 41 or 42 41 wherein said substantially vertical axis comprises a mast.
vertical is adapted to support a dental instrument selected from the group consisting of

**45.** A dental support system comprising:
a substantially horizontal shaft rotatably supporting a yoke member;
a substantially vertical shaft rotatably positioning a pivot member; and
a brake device adapted to damp a rotation of said pivot member about said substantially vertical shaft.

**46.** The dental support system of claim 45 wherein said brake device comprises a frictional member.

**47.** The dental support system of claim 46 wherein said frictional member comprises material selected from the group consisting of a synthetic elastomeric polymer, a natural elastomeric polymer, a viscous fluid and combinations thereof.

**48.** The dental support system of claim 45 wherein said brake device comprises a compression plug adapted to urge a first friction surface of said frictional member against a second frictional surface.

**49.** The dental support system of claim 48 wherein said compression plug comprises a substantially cylindrical outer surface bearing a plurality of threads.

**50.** The dental support system of claim 45 wherein said brake device further comprises a spring to urge the first friction surface of said frictional member against the second frictional surface.

**51.** The dental support system of claim 1 wherein said dental instrument comprises a dental whitening system, a dental curing system, a dental examination system, a viewing and cleaning instrument; an imaging equipment; an X-ray equipment, a root canal apex locator, or combinations thereof.
